# EUROPEAN PATENT SPECIFICATION

(11) **EP 4 746 866 B1**
(45) Date of publication and mention of the grant of the patent: **18.11.2026**
(21) Application number: 25771020.2
(22) Date of filing: 17.09.2025
(51) Int. Cl.: A61K 31/20, A61K 31/397, A61K 31/40, A61K 31/505, A61K 31/506, A61K 45/06, A61P 9/04, A61P 9/10

(54) **OBICETRAPIB FOR USE IN METHODS FOR TREATING A SUBJECT HAVING HETEROZYGOUS FAMILIAL HYPERCHOLESTEROLEMIA (HEFH) AND/OR ATHEROSCLEROTIC CARDIOVASCULAR DISEASE (ASCVD)**
OBICETRAPIB ZUR VERWENDUNG IN VERFAHREN ZUR BEHANDLUNG EINER PERSON MIT HETEROZYGOTER FAMILIÄRER HYPERCHOLESTEROLÄMIE (HEFH) UND/ODER ATHEROSKLEROTISCHER KARDIOVASKULÄRER ERKRANKUNG (ASCVD)
OBICETRAPIB DESTINÉ À ÊTRE UTILISÉ DANS DES MÉTHODES DE TRAITEMENT D'UN SUJET AYANT UNE HYPERCHOLESTÉROLÉMIE FAMILIALE HÉTÉROZYGOTE (HEFH) ET/OU UNE MALADIE CARDIOVASCULAIRE ATHÉROSCLÉREUSE (ASCVD)

(30) Priority: 17.09.2024 US 202463695847 P; 09.12.2024 US 202463729799 P; 04.03.2025 US 202563766906 P
(43) Date of publication of application: 27.05.2026
(60) Divisional application: 26209923.7
(73) Proprietor: NewAmsterdam Pharma B.V., 1411 DC Naarden (NL)
(72) Inventor: DAVIDSON, Michael Harvey, 1411 DC Naarden (NL); DITMARSCH, Marc, 1411 DC Naarden (NL); KASTELEIN, Johannes Jacob Pieter, 1411 DC Naarden (NL); KLING, Douglas Fitzgerald, 1411 DC Naarden (NL)
(74) Representative: Nederlandsch Octrooibureau
(86) International application number: PCT/EP2025/076528
(87) International publication number: WO 2026/062050

(56) References cited:
- WO-A1-2023/006657
- NICHOLLS STEPHEN J ET AL: "Obicetrapib on top of maximally tolerated lipid-modifying therapies in participants with or at high risk for atherosclerotic cardiovascular disease: rationale and designs of BROADWAY and BROOKLYN", AMERICAN HEART JOURNAL, ELSEVIER, AMSTERDAM, NL, vol. 274, 4 May 2024 (2024-05-04), pages 32 - 45, XP087547721, ISSN: 0002-8703, [retrieved on 20240504], DOI: 10.1016/J.AHJ.2024.05.002
- ANONYMOUS: "Randomized Study to Evaluate the Effect of Obicetrapib on Top of Maximum Tolerated Lipid-Modifying Therapies (BROADWAY) - NCT05142722", 3 July 2024 (2024-07-03), XP093337250, Retrieved from the Internet <URL:https://clinicaltrials.gov/study/NCT05142722?tab=history&a=9#version-content-panel>
- NURMOHAMED NICK S ET AL: "Cholesteryl ester transfer protein inhibitors: from high-density lipoprotein cholesterol to low-density lipoprotein cholesterol lowering agents?", CARDIOVASCULAR RESEARCH, vol. 118, no. 14, 26 November 2021 (2021-11-26), England, pages 2919 - 2931, XP093268413, ISSN: 0008-6363, Retrieved from the Internet <URL:https://watermark.silverchair.com/cvab350.pdf?token=AQECAHi208BE49Ooan9kkhW_Ercy7Dm3ZL_9Cf3qfKAc485ysgAAA3kwggN1BgkqhkiG9w0BBwagggNmMIIDYgIBADCCA1sGCSqGSIb3DQEHATAeBglghkgBZQMEAS4wEQQMoel_Hlg-4HtCW7_RAgEQgIIDLF1B_RKxTH_RaZETVzrCHlRqAZz7tzvKY7RR1yIf66P5LFJsAC56PgBdELpAjPHQjo6fk20CuaCVxYjIIMTz5eQ_gDEh> DOI: 10.1093/cvr/cvab350

## Description

### 21. CROSS-REFERENCE TO RELATED APPLICATIONS

This application claims the benefit of and priority to U.S. Provisional Application Nos. 63/695,847, filed on September 17, 2024, 63/729,799, filed on December 9, 2024, and 63/766,906, filed on March 4, 2025,

### 2. BACKGROUND

Elevated low-density lipoprotein cholesterol (LDL-C) is a major modifiable risk factor for the development of cardiovascular disease (CVD). Lowering LDL-C has been shown to reduce the risk of cardiovascular (CV) events, and the risk reduction is linearly proportional to the absolute LDL-C reduction. Lowering LDL-C is the primary therapeutic lipid target in atherosclerotic cardiovascular disease (ASCVD) and heterozygous familial hypercholesterolemia (HeFH) patients. Intensive LDL-C lowering relative to modest reduction confers a greater benefit in patients at high CV risk.

Statins are generally the drug of first choice in treating dyslipidemia. Statins are considered first line therapy for reducing LDL-C levels. However, two thirds of ASCVD and HeFH patients do not achieve acceptable levels of LDL-C with statins alone, even with high-intensity statin therapy (HIS). Therefore, there is an unmet need in clinical practice for safe and efficacious pharmacological treatment modalities for patients who require additional lowering of LDL-C.

According to Nurmohamed et al ("Cholesteryl ester transfer protein inhibitors: from high-density lipoprotein cholesterol to low-density lipoprotein cholesterol lowering agents", CARDIOVASCULAR RESEARCH, vol. 118, no. 14, 26 November 2021 pages 2919-2931), cholesteryl ester transfer protein (CETP) inhibition may reduce major cardiovascular events by lowering LDL-C and the CETP inhibitor has shown a 45% LDL-C reduction in a phase 2 trial. Reductions in MACE as a result of CETP treatment are not disclosed.

There is an unmet need in clinical practice for safe and efficacious pharmacological treatment modalities for treating heterozygous familial hypercholesterolemia (HeFH) and/or atherosclerotic cardiovascular disease (ASCVD).

### 3. SUMMARY OF THE INVENTION

Generally stated, the present disclosure relates to methods of treating heterozygous familial hypercholesterolemia (HeFH) and/or atherosclerotic cardiovascular disease (ASCVD) in a subject are provided. The method comprises administering to the subject a pharmaceutical composition comprising obicetrapib or a pharmaceutically acceptable salt, hydrate, or solvate thereof.

In one aspect, a pharmaceutical composition comprising obicetrapib or a pharmaceutically acceptable salt, hydrate, or solvate thereof is provided, for use in a methods are of reducing the risk and/or incidence of a major adverse cardiovascular event (MACE) event in 3-composite MACE (MACE3) or in 4-compositie MACE (MACE4) in a subject having heterozygous familial hypercholesterolemia (HeFH) and/or atherosclerotic cardiovascular disease (ASCVD), the method comprising:
administering to the subject a therapeutically effective amount of a pharmaceutical composition comprising obicetrapib or a pharmaceutically acceptable salt, hydrate, or solvate thereof, for at least 6 months, as defined in the claims.

In certain embodiments, the method reduces the risk of MACE3, where MACE3 comprises coronary heart disease death, nonfatal myocardial infarction and coronary revascularization.

In various embodiments, the method reduces the risk of MACE4, where MACE4 comprises coronary heart disease death, nonfatal myocardial infarction, ischemic stroke and coronary revascularization.

In various embodiments, the presence of HeFH is determined by genotyping.

In various embodiments, the presence of HeFH is determined by clinical assessment.

In certain embodiments, the presence of ASCVD is determined by the presence of at least one of the conditions selected from the group consisting of: coronary artery disease, cerebrovascular disease, and peripheral arterial disease.

In certain embodiments, the subject, prior to first administration of obicetrapib, has a fasting serum LDL-C equal to or greater than 70 mg/dL (1.81 mmol/L).

In certain embodiments, the subject, prior to first administration of obicetrapib, is on maximally tolerated lipid-modifying therapy.

In various embodiments, the maximally tolerated lipid-modifying therapy comprises at least one treatment selected from the group consisting of: a statin at a maximally tolerated stable dose, ezetimibe, bempedoic acid, a proprotein convertase subtilisin/kexin type 9 (PCSK9)-targeted therapy, a fibrate other than gemfibrozil, and combinations thereof.

In some embodiments, the subject, prior to first administration of obicetrapib, is on high-intensity statin therapy (HIS).

In various embodiments, the high-intensity statin therapy (HIS) is atorvastatin at 40 mg or 80 mg.

In some embodiments, the high-intensity statin therapy (HIS) is rosuvastatin at 20 mg or 40 mg.

In various embodiments, the subject prior to the first administration of obicetrapib, is not diabetic.

In various embodiments, the subject prior to the first administration of obicetrapib, is normoglycemic.

In certain embodiments, the subject prior to the first administration of obicetrapib, is diabetic.

In various embodiments, obicetrapib or a pharmaceutically acceptable salt, hydrate, or solvate thereof, is administered orally at a dose of 10 mg once daily.

In various embodiments, obicetrapib or a pharmaceutically acceptable salt, hydrate, or solvate thereof is administered daily for at least 6 months, 7 months, 8 months, 9 months, 10 months, 11 months, 12 months, 13 months, 14 months, 15 months, 16 months, 17 months, 18 months, 19 months, 20 months, 21 months, 22 months, 23 months, or 24 months.

In certain embodiments, a calcium salt of obicetrapib is administered to the subject. In various embodiments, obicetrapib hemicalcium is administered to the subject. In certain embodiments, amorphous obicetrapib hemicalcium is administered to the subject.

In certain embodiments, the pharmaceutical composition is in a solid dosage form. In certain embodiments, the solid dosage form is a tablet. In certain embodiments, the tablet further comprises a film coating.

### 4. BRIEF DESCRIPTION OF THE DRAWINGS

These and other features, aspects, and advantages of the present invention will become better understood with regard to the following description, and accompanying drawings, where:

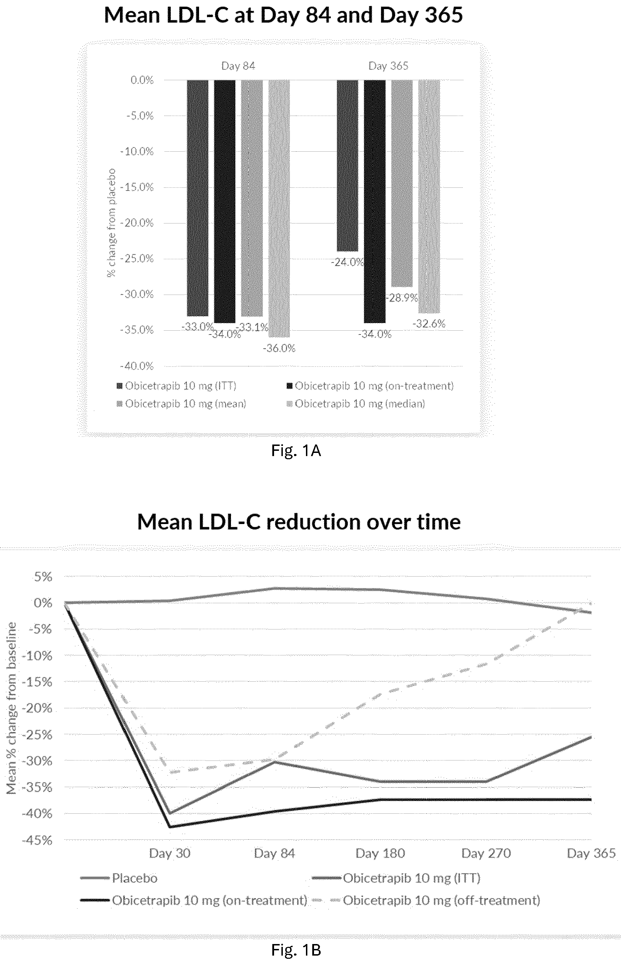

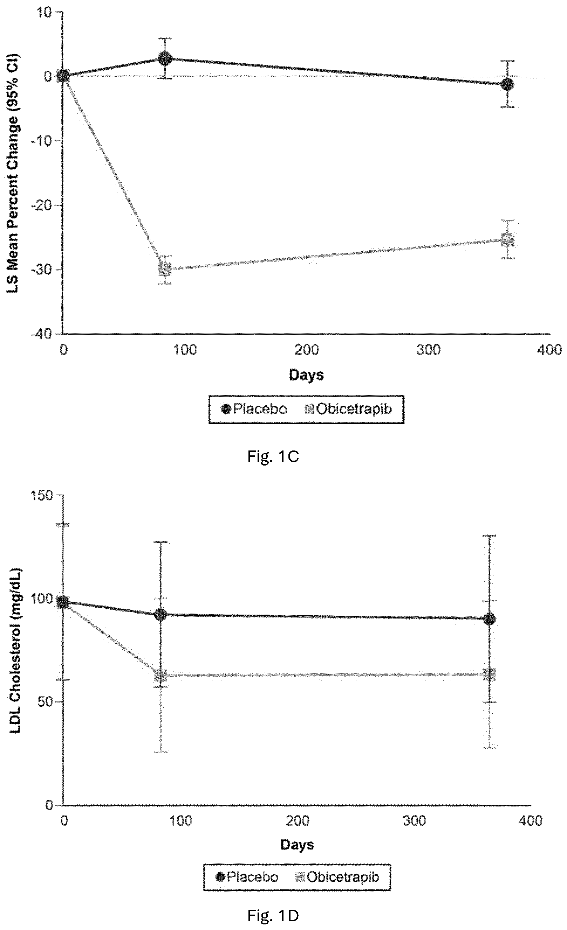

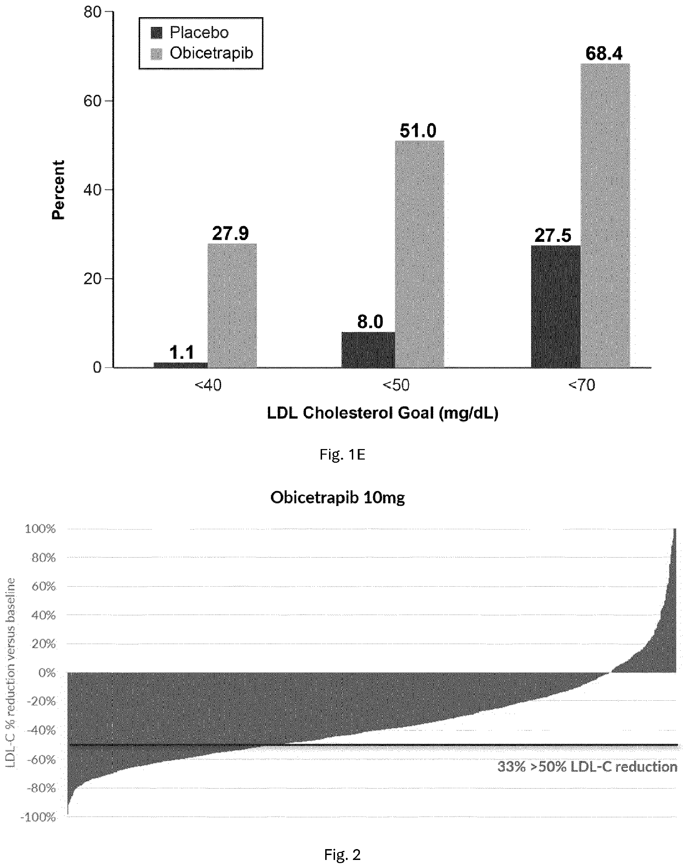

FIGs. 1A-1E shows placebo-adjusted change in LDL cholesterol (LDL-C) data observed in the trial populations. FIG. 1A shows placebo-adjusted LDL-C reductions from baseline observed at Day 84 and at Day 365 for obicetrapib 10 mg in the intention-to-treat population (ITT), in the on-treatment population, as well as placebo-adjusted mean and median reductions from baseline for obicetrapib across the obicetrapib-treated population. FIG. 1B graphs LDL-C reduction from baseline observed at various time points over the course of the study for obicetrapib 10 mg in the intention-to-treat population, the on-treatment population, and the off-treatment population versus placebo. FIG. 1C graphs least squares (LS) mean percent changes in LDL-C in subjects treated with obicetrapib and placebo subjects (whiskers indicate the 95% confidence interval (CI)) to Day 365. FIG. 1D is a line graph showing absolute achieved levels of LDL-C in populations receiving obicetrapib and in placebo subjects from Day 0 to Day 365. FIG. 1E is a bar graph showing percentage of subjects achieving an LDL-C less than 40, 55 and 70 mg/dL in subjects treated with obicetrapib or in the placebo group.
FIG. 2 is a waterfall analysis for LDL-C reduction versus baseline for obicetrapib 10 mg at Day 84.
FIGs. 3A-3B are bar graphs showing the percentage of subjects achieving LDL-C levels of less than 55 mg/dL and less than 70 mg/dL at Day 84 and Day 365 for placebo and obicetrapib 10 mg groups. FIG. 3A shows the data for Day 84. FIG. 3B shows the data for Day 365.
FIGs. 4A-4D are line graphs showing LS mean percent changes from baseline over time in select lipid and lipoprotein parameters separately in subjects treated with obicetrapib and those in the placebo group. FIG. 4A is a line graph showing the percent change in apolipoprotein B (apoB) (95% CI). FIG. 4B is a line graph showing the percent change in non-HDL cholesterol (95% CI). FIG. 4C is a line graph showing the percent change in HDL cholesterol (95% CI). FIG. 4D is a line graph showing the percent change in triglycerides (TG) (95% CI).
FIG. 5 is Kaplan-Meier plot of time to the first occurrence of a major adverse cardiovascular event (MACE) in the intention-to-treat (ITT) population of obicetrapib 10 mg versus placebo, showing clear separation of the curves after about 200 days of daily treatment with 10 mg obicetrapib.
FIG. 6 summarizes enrollment information for two phase III clinical trial studies, BROOKLYN (upper panel) and BROADWAY (lower panel).
FIG. 7 is a bar graph showing the effect of obicetrapib on mean hemoglobin A1c (HbA1c) at day 365 in pooled BROOKLYN and BROADWAY studies in (a) all patients without diabetes at baseline, (b) normoglycemic patients at baseline, and (c) prediabetic patients at baseline.
FIG. 8 is a bar graph showing the effect of obicetrapib on development of new-onset diabetes at day 365 in pooled BROOKLYN and BROADWAY (a) all patients without diabetes at baseline, normoglycemic at baseline, or prediabetic at baseline.
FIG. 9 is a line graph showing cumulative incidence of composite coronary heart disease death, nonfatal myocardial infarction, ischemic stroke and coronary revascularization (4-component MACE) data in pooled BROOKLYN and BROADWAY patients treated with placebo or obicetrapib.
FIG. 10 is a line graph showing cumulative incidence of composite coronary heart disease death, nonfatal myocardial infarction, and coronary revascularization (3-component MACE) data in pooled BROOKLYN and BROADWAY patients treated with placebo or obicetrapib.
FIG. 11 is a line graph showing cumulative incidence of composite coronary heart disease death, nonfatal myocardial infarction, ischemic stroke and coronary revascularization (4-component MACE) data in pooled BROOKLYN and BROADWAY patients treated with placebo or obicetrapib, with data from months 0-6 (left panel) and data from months 6-15 (right panel) plotted separately.
FIG. 12 is a line graph showing cumulative incidence of composite coronary heart disease death, nonfatal myocardial infarction, and coronary revascularization (3-component MACE) data in pooled BROOKLYN and BROADWAY patients treated with placebo or obicetrapib, with data from months 0-6 (left panel) and data from months 6-15 (right panel) plotted separately.
FIG. 13A-13D show time-weighted levels of achieved levels lipid biomarkers and the 12-month incidence of 4-component MACE with 95% confidence intervals (CI). Analyses were not mutually adjusted for other lipid and lipoprotein levels, given the relatively small number of events observed. Knots are at the 25th, 50th, and 75th percentiles and are plotted from the approximate 5th and 95th percentiles of time-weighted biomarker. FIG. 13A shows time-weighted LDL-C levels. FIG. 13B shows time-weighted ApoB levels. FIG. 13C shows time-weighted Lp(a). FIG. 13D shows time-weighted HDL-C.

### 5. DETAILED DESCRIPTION OF THE INVENTION

### 5.1. Definitions

Unless defined otherwise, all technical and scientific terms used herein have the meaning commonly understood by one of ordinary skill in the art to which the invention pertains.

The term **"subject"** refers to a human or non-human mammal, including, but not limited to, bovine, equine, canine, ovine, feline, and rodent, including murine and rattus, subjects. A **"patient"** is a human subject in need of treatment.

As used herein, the terms **"treat," "treating," "treatment,"** and the like refer to reducing or ameliorating a disorder, and/or signs or symptoms associated therewith, or slowing or halting the progression thereof. It will be appreciated that, although not precluded, treating a disorder or condition does not require that the disorder, condition or symptoms associated therewith be completely eliminated.

As used herein, **"pre-treatment"** means prior to the first administration of obicetrapib according to the methods described herein. Pre-treatment does not exclude, and often includes, the prior administration of treatments other than obicetrapib.

As used herein, **"post-treatment"** means after the administration of obicetrapib according to the methods described herein. Post-treatment includes after any administration of obicetrapib at any dosage described herein. Post-treatment also includes after the treatment phase of obicetrapib.

In this disclosure, **"comprises," "comprising," "containing," "having," "includes," "including,"** and linguistic variants thereof have the meaning ascribed to them in U.S. Patent law, permitting the presence of additional components beyond those explicitly recited.

The term **"biological sample"** refers to any tissue, cell, fluid, or other material derived from an organism (e.g., human subject). In certain embodiments, the biological sample is serum, plasma, or blood.

### 5.2. Methods of Treating Heterozygous Familial Hypercholesterolemia (HeFH) and/or Atherosclerotic Cardiovascular Disease (ASCVD)

### 5.2.1. HeFH and/or ASCVD

Generally, the present disclosure relates to methods of treating heterozygous familial hypercholesterolemia (HeFH) and/or atherosclerotic cardiovascular disease (ASCVD) in a subject. The method comprises administering to the subject a therapeutically effective amount of a pharmaceutical composition comprising obicetrapib or a pharmaceutically acceptable salt, hydrate, or solvate thereof.

In some embodiments, the presence of HeFH is determined by genotyping. In some embodiments, HeFH is determined by clinical assessment.

In some embodiments, the presence of ASCVD is determined by the presence of at least one of the conditions selected from the group consisting of: coronary artery disease, cerebrovascular disease, and peripheral arterial disease.

In some embodiments, subject is at risk for development of accelerated vascular disease, heart attack and/or stroke. In some embodiments the subject is at risk for coronary artery disease (CAD). In some embodiments, the increased risk of development is within 1-9 years. In some embodiments, the increased risk of development is within 1-8 years. In some embodiments, the increased risk of development is within 1-7 years. In some embodiments, the increased risk of development is within 1-6 years. In some embodiments, the increased risk of development is within 1-5 years. In some embodiments, the increased risk of development is within 1-4 years. In some embodiments, the increased risk of development is within 4-10 years. In some embodiments, the increased risk of development is within 4-9 years. In some embodiments, the increased risk of development is within 4-8 years. In some embodiments, the increased risk of development is within 5-7 years. In some embodiments, the increased risk of development is within 4-6 years.

In some embodiments the subject is at least 30, 35, 40, 45, 50, 55, 60, 65, or 70 years old.

In another embodiment, the subject is a subjects with HeFH and/or ASCVD who is not adequately controlled by their lipid-modifying therapies are provided. The methods comprise orally administering 10 mg obicetrapib once per day for at least 6 months.

In some embodiments, methods reduce the incidence of MACE in 3-composite MACE. In some embodiments, the composite consists of coronary heart disease death, nonfatal myocardial infarction and coronary revascularization.

In some embodiments, the methods reduce the incidence of MACE in 4-compose MACE. In some embodiments, the composite consists of coronary heart disease death, nonfatal myocardial infarction, ischemic stroke and coronary revascularization.

In some embodiments, obicetrapib is administered in combination with concomitant lipid-modifying therapies. In some embodiments, those concomitant lipid modifying therapies include administration of at least one of statins, high intensity statins, PCSK9 inhibitors, and ezetimibe.

### 5.2.2. Dose Regimen

In various embodiments, obicetrapib or pharmaceutically acceptable salt thereof is administered orally. Whenever a salt is administered, the dose is expressed as the amount of the obicetrapib anion within the salt.

In typical embodiments, obicetrapib is administered as a tablet for oral administration. In various embodiments, the dose of obicetrapib or salt thereof is 2.5-25 mg by mouth per day (2.5-25 mg po QD). In some embodiments, the dose of obicetrapib or salt thereof is 5-20 mg by mouth per day (5-20 mg po QD). In some embodiments, the dose of obicetrapib or salt thereof is 10-20 mg by mouth per day (10-20 mg po QD). In some specific embodiments, the dose of obicetrapib or salt thereof is 2.5 mg po QD, 3.0 mg po QD, 3.5 mg po QD, 4.0 mg po QD, 4.5 mg po QD, 5 mg po QD, 5.5 mg po QD, 6 mg po QD, 6.5 mg po QD, 7 mg po QD, 7.5 mg po QD, 8 mg po QD, 8.5 mg po QD, 9 mg po QD, 9.5 mg po QD, 10 mg po QD, 10.5 mg po QD, 11 mg po QD, 11.5 mg po QD, 12 mg po QD, 12.5 mg po QD, 13 mg po QD, 13.5 mg po QD, 14 mg po QD, 14.5 mg po QD, 15 mg po QD, 15.5 mg po QD, 16 mg po QD, 16.5 mg po QD, 17 mg po QD, 17.5 mg po QD, 18 mg po QD, 18.5 mg po QD, 19 mg po QD, 19.5 mg po QD, 20 mg po QD, 25 mg po QD or 30 mg po QD.

In some specific embodiments, the dose of obicetrapib or salt thereof is about 2.5 mg po QD, 3.0 mg po QD, 3.5 mg po QD, 4.0 mg po QD, 4.5 mg po QD, 5 mg po QD, 5.5 mg po QD, 6 mg po QD, 6.5 mg po QD, 7 mg po QD, 7.5 mg po QD, 8 mg po QD, 8.5 mg po QD, 9 mg po QD, 9.5 mg po QD, 10 mg po QD, 10.5 mg po QD, 11 mg po QD, 11.5 mg po QD, 12 mg po QD, 12.5 mg po QD, 13 mg po QD, 13.5 mg po QD, 14 mg po QD, 14.5 mg po QD, 15 mg po QD, 15.5 mg po QD, 16 mg po QD, 16.5 mg po QD, 17 mg po QD, 17.5 mg po QD, 18 mg po QD, 18.5 mg po QD, 19 mg po QD, 19.5 mg po QD, 20 mg po QD, 25 mg po QD or 30 mg po QD.

In some specific embodiments, the daily dose of obicetrapib or salt thereof is 10.0, 10.1, 10.2, 10.3, 10.4, 10.5, 10.6, 10.7, 10.8, 10.9, 11.0, 11.1, 11.2, 11.3, 11.4, 11.5, 11.6, 11.7, 11.8, 11.9, 12.0, 12.1, 12.2, 12.3, 12.4, 12.5, 12.6, 12.7, 12.8, 12.9, 13.0, 13.1, 13.2, 13.3, 13.4, 13.5, 13.6 13.7, 13.8, 13.9, 14.0, 14.1, 14.2, 14.3, 14.4, 14.5, 14.6, 14.7, 14.8, 14.9, 15.0, 15,1, 15.2, 15.3, 15.4, 15.5, 15.6, 15.7, 15.8, 15.9, 16.0, 16.1, 16.2, 16.3, 16.4, 16.5, 16.6, 16.7, 16.8, 16.9, 17.0, 17.1, 17.2, 17.3, 17.4, 17.5, 17.6, 17.7, 17.8, 17.9, 18.0, 18.1, 18.2, 18.3, 18.4. 18.5, 18.6, 18.7, 18.8, 18.9, 19.0, 19.1, 19.2, 19.3, 19.4, 19.5, 19.6, 19.7, 19.8, 19.9, 20.0, 20.1, 20.2, 20.3, 20.4, 20.5, 20.6, 20.7, 20.8, 20.9, 21.0, 21.1, 21.2, 21.3, 21.4, 21.5, 21.6, 21.7, 21.8, 21.9, 22.0, 22.1, 22.2, 22.3, 22.4, 22.5, 22.6, 22.7, 22.8, 22.9, 23.0, 23.1, 23.2, 23.3, 23.4, 23.5, 23.6, 23.7, 23.8, 23.9. 24.0, 24.1, 24.2, 24.3, 24.4, 24.5, 24.6, 24.7, 24.8, 24.9, 25.0, 25.1, 25.2, 25.3, 25.4, 25.5, 25.6, 25.7, 25.8, 25.9, 26.0, 26.1, 26.2, 26.3, 26.4, 26.5. 26.6, 26.7, 26.8, 26.9, 27.0, 27.1, 27.2, 27.3, 27.4, 27.5, 27.6, 27.7, 27.8, 27.9, 28.0, 28.1, 28.2, 28.3, 28.4, 28.5, 28.6, 28.7, 28.8, 28.9, 29.0, 29.1, 29.2, 29.3, 29.4, 29.5, 29.6, 29.7, 29.8, 29.9, or 30.0 mg.

In some specific embodiments, the dose of obicetrapib or salt thereof is 10.0, 10.1, 10.2, 10.3, 10.4, 10.5, 10.6, 10.7, 10.8, 10.9, 11.0, 11.1, 11.2, 11.3, 11.4, 11.5, 11.6, 11.7, 11.8, 11.9, 12.0, 12.1, 12.2, 12.3, 12.4, 12.5, 12.6, 12.7, 12.8, 12.9, 13.0, 13.1, 13.2, 13.3, 13.4, 13.5, 13.6 13.7, 13.8, 13.9, 14.0, 14.1, 14.2, 14.3, 14.4, 14.5, 14.6, 14.7, 14.8, 14.9, 15.0, 15,1, 15.2, 15.3, 15.4, 15.5, 15.6, 15.7, 15.8, 15.9, 16.0, 16.1, 16.2, 16.3, 16.4, 16.5, 16.6, 16.7, 16.8, 16.9, 17.0, 17.1, 17.2, 17.3, 17.4, 17.5, 17.6, 17.7, 17.8, 17.9, 18.0, 18.1, 18.2, 18.3, 18.4. 18.5, 18.6, 18.7, 18.8, 18.9, 19.0, 19.1, 19.2, 19.3, 19.4, 19.5, 19.6, 19.7, 19.8, 19.9, 20.0, 20.1, 20.2, 20.3, 20.4, 20.5, 20.6, 20.7, 20.8, 20.9, 21.0, 21.1, 21.2, 21.3, 21.4, 21.5, 21.6, 21.7, 21.8, 21.9, 22.0, 22.1, 22.2, 22.3, 22.4, 22.5, 22.6, 22.7, 22.8, 22.9, 23.0, 23.1, 23.2, 23.3, 23.4, 23.5, 23.6, 23.7, 23.8, 23.9. 24.0, 24.1, 24.2, 24.3, 24.4, 24.5, 24.6, 24.7, 24.8, 24.9, 25.0, 25.1, 25.2, 25.3, 25.4, 25.5, 25.6, 25.7, 25.8, 25.9, 26.0, 26.1, 26.2, 26.3, 26.4, 26.5. 26.6, 26.7, 26.8, 26.9, 27.0, 27.1, 27.2, 27.3, 27.4, 27.5, 27.6, 27.7, 27.8, 27.9, 28.0, 28.1, 28.2, 28.3, 28.4, 28.5, 28.6, 28.7, 28.8, 28.9, 29.0, 29.1, 29.2, 29.3, 29.4, 29.5, 29.6, 29.7, 29.8, 29.9, or 30.0 mg po QD.

In various embodiments, the dose is administered once per day. In some embodiments, the dose is divided and the 2.5-25 mg total daily dose, or 5-20 mg total daily dose, or 10-20 mg total daily dose, is administered as a plurality of divided doses.

In various embodiments, obicetrapib is administered as a tablet. In some embodiments, the tablet comprises 2.5 mg, 5 mg, 7.5 mg, 10 mg, 15 mg, 20 mg or 25 mg of obicetrapib or pharmaceutically acceptable salt thereof. In some embodiments, the tablet contains obicetrapib as the calcium salt. In particular embodiments, the table contains 5 mg obicetrapib as a calcium salt.

In specific embodiments, tablets are round, 6 mm in diameter, white film-coated tablets, containing 5 mg of obicetrapib as the calcium salt. In specific embodiments, tablets are round, 6 mm in diameter, white film-coated tablets, containing 10 mg of obicetrapib as the calcium salt. In specific embodiments, the excipients present in the tablet cores are microcrystalline cellulose, mannitol, sodium starch glycollate, colloidal silicon dioxide, and magnesium stearate. In specific embodiments, a commercially available film-coating formula (Opadry II white, ex Colorcon) is applied to the cores.

In various embodiments, obicetrapib or salt thereof is administered once daily for at least 8 weeks, at least 6 months, at least 12 months, at least 24 months, or at least 36 months.

In some embodiments obicetrapib is amorphous obicetrapib hemicalcium.

In some embodiments, the subject, prior to first administration of obicetrapib, is on maximally tolerated lipid-modifying therapy. In some of these embodiments, the maximally tolerated lipid-modifying therapy is selected from the group consisting of: a statin at a maximally tolerated stable dose, ezetimibe, bempedoic acid, a proprotein convertase subtilisin/kexin type 9 (PCSK9)-targeted therapy, a fibrate, provided that the fibrate is not gemfibrozil, and combinations thereof. In certain embodiments, the subject, prior to first administration of obicetrapib, is on high-intensity statin therapy (HIS). In some embodiments, the high-intensity statin therapy (HIS) is atorvastatin at 40 mg or 80 mg. In some embodiments, the high-intensity statin therapy (HIS) is rosuvastatin at 20 mg or 40 mg.

In some embodiments, the subject, prior to first administration of obicetrapib, has a fasting serum LDL-C equal to or greater than 70 mg/dL (1.8 mmol/L). In some embodiments, the subject, prior to first administration of obicetrapib, has a fasting serum TG less than 400 mg/dL (4.5 mmol/L). In some embodiments, the subject, prior to first administration of obicetrapib, has an estimated glomerular filtration rate (eGFR) equal to or greater than 30 mL/min/1.73 m2, calculated using the Chronic Kidney Disease Epidemiology Collaboration equation.

In some embodiments, administering the pharmaceutical composition reduces the serum level of LDL-C as compared to pre-treatment levels. In some embodiments, administering the pharmaceutical composition reduces the serum level of ApoB as compared to pre-treatment levels. In some embodiments, administering the pharmaceutical composition reduces the serum level of non-high density lipoprotein cholesterol (non-HDL-C) as compared to pre-treatment levels. In some embodiments, administering the pharmaceutical composition reduces the serum level of total cholesterol (TC) as compared to pre-treatment levels. In some embodiments, administering the pharmaceutical composition reduces the serum level of Lp(a) as compared to pre-treatment levels. In some embodiments, administering the pharmaceutical composition reduces the serum level of triglycerides (TG) as compared to pre-treatment levels.

In some embodiments, administering the pharmaceutical composition reduces the fasting serum level of LDL-C as compared to pre-treatment levels. In some embodiments, administering the pharmaceutical composition reduces the fasting serum level of ApoB as compared to pre-treatment levels. In some embodiments, administering the pharmaceutical composition reduces the fasting serum level of non-HDL-C as compared to pre-treatment levels. In some embodiments, administering the pharmaceutical composition reduces the fasting serum level of total cholesterol (TC) as compared to pre-treatment levels. In some embodiments, administering the pharmaceutical composition reduces the fasting serum level of Lp(a) as compared to pre-treatment levels. In some embodiments, administering the pharmaceutical composition reduces the fasting serum level of triglycerides (TG) as compared to pre-treatment levels.

In some embodiments, administering the pharmaceutical composition reduces the HbA1c level as compared to pre-treatment levels. In some embodiments, administering the pharmaceutical composition reduces the HOMA-IR level as compared to pre-treatment levels. In some embodiments, administering the pharmaceutical composition reduces the blood glucose level as compared to pre-treatment levels. In some embodiments the reductions following administration of the pharmaceutical composition compared to pre-treatment levels is 60%, 59%, 58%, 57%, 56%, 55%, 54%, 53%, 52%, 51%, 50%, 49%, 48%, 47%, 46%, 45%, 44%, 43%, 42%, 41%, 40%, 39%, 38%, 37%, 36%, 35%, 34%, 33%, 32%, 31%, 30%, 29%, 28%, 27%, 26%, 25%, 24%, 23%, 22%, 21%, 20%, 19%, 18%, 17%, 16%, 15%, 14%, 13%, 12%, 11%, 10%, 9%, 8%, 7%, 6%, or 5%.

In some embodiments the reductions following administration of the pharmaceutical composition compared to pre-treatment levels is about 60%, 59%, 58%, 57%, 56%, 55%, 54%, 53%, 52%, 51%, 50%, 49%, 48%, 47%, 46%, 45%, 44%, 43%, 42%, 41%, 40%, 39%, 38%, 37%, 36%, 35%, 34%, 33%, 32%, 31%, 30%, 29%, 28%, 27%, 26%, 25%, 24%, 23%, 22%, 21%, 20%, 19%, 18%, 17%, 16%, 15%, 14%, 13%, 12%, 11%, 10%, 9%, 8%, 7%, 6%, or 5%.

In some embodiments, the reductions in levels of LDL-C following administration of the pharmaceutical composition as compared to pre-treatment LDL-C levels is 60%, 59%, 58%, 57%, 56%, 55%, 54%, 53%, 52%, 51%, 50%, 49%, 48%, 47%, 46%, 45%, 44%, 43%, 42%, 41%, 40%, 39%, 38%, 37%, 36%, 35%, 34%, 33%, 32%, 31%, 30%, 29%, 28%, 27%, 26%, 25%, 24%, 23%, 22%, 21%, 20%, 19%, 18%, 17%, 16%, 15%, 14%, 13%, 12%, 11%, 10%, 9%, 8%, 7%, 6%, or 5%.

In some embodiments, the reduction is about 60%, 59%, 58%, 57%, 56%, 55%, 54%, 53%, 52%, 51%, 50%, 49%, 48%, 47%, 46%, 45%, 44%, 43%, 42%, 41%, 40%, 39%, 38%, 37%, 36%, 35%, 34%, 33%, 32%, 31%, 30%, 29%, 28%, 27%, 26%, 25%, 24%, 23%, 22%, 21%, 20%, 19%, 18%, 17%, 16%, 15%, 14%, 13%, 12%, 11%, 10%, 9%, 8%, 7%, 6%, or 5%.

In some embodiments, the reductions in levels of ApoB following administration of the pharmaceutical composition as compared to pre-treatment ApoB levels is 60%, 59%, 58%, 57%, 56%, 55%, 54%, 53%, 52%, 51%, 50%, 49%, 48%, 47%, 46%, 45%, 44%, 43%, 42%, 41%, 40%, 39%, 38%, 37%, 36%, 35%, 34%, 33%, 32%, 31%, 30%, 29%, 28%, 27%, 26%, 25%, 24%, 23%, 22%, 21%, 20%, 19%, 18%, 17%, 16%, 15%, 14%, 13%, 12%, 11%, 10%, 9%, 8%, 7%, 6%, or 5%.

In some embodiments, the reduction is about 60%, 59%, 58%, 57%, 56%, 55%, 54%, 53%, 52%, 51%, 50%, 49%, 48%, 47%, 46%, 45%, 44%, 43%, 42%, 41%, 40%, 39%, 38%, 37%, 36%, 35%, 34%, 33%, 32%, 31%, 30%, 29%, 28%, 27%, 26%, 25%, 24%, 23%, 22%, 21%, 20%, 19%, 18%, 17%, 16%, 15%, 14%, 13%, 12%, 11%, 10%, 9%, 8%, 7%, 6%, or 5%.

In some embodiments, the reductions in levels of non-HDL-C following administration of the pharmaceutical composition as compared to pre-treatment non-HDL-C levels is 60%, 59%, 58%, 57%, 56%, 55%, 54%, 53%, 52%, 51%, 50%, 49%, 48%, 47%, 46%, 45%, 44%, 43%, 42%, 41%, 40%, 39%, 38%, 37%, 36%, 35%, 34%, 33%, 32%, 31%, 30%, 29%, 28%, 27%, 26%, 25%, 24%, 23%, 22%, 21%, 20%, 19%, 18%, 17%, 16%, 15%, 14%, 13%, 12%, 11%, 10%, 9%, 8%, 7%, 6%, or 5%.

In some embodiments, the reduction is about 60%, 59%, 58%, 57%, 56%, 55%, 54%, 53%, 52%, 51%, 50%, 49%, 48%, 47%, 46%, 45%, 44%, 43%, 42%, 41%, 40%, 39%, 38%, 37%, 36%, 35%, 34%, 33%, 32%, 31%, 30%, 29%, 28%, 27%, 26%, 25%, 24%, 23%, 22%, 21%, 20%, 19%, 18%, 17%, 16%, 15%, 14%, 13%, 12%, 11%, 10%, 9%, 8%, 7%, 6%, or 5%.

In some embodiments, the reductions in levels of total cholesterol (TC) following administration of the pharmaceutical composition as compared to pre-treatment total cholesterol (TC) levels is 60%, 59%, 58%, 57%, 56%, 55%, 54%, 53%, 52%, 51%, 50%, 49%, 48%, 47%, 46%, 45%, 44%, 43%, 42%, 41%, 40%, 39%, 38%, 37%, 36%, 35%, 34%, 33%, 32%, 31%, 30%, 29%, 28%, 27%, 26%, 25%, 24%, 23%, 22%, 21%, 20%, 19%, 18%, 17%, 16%, 15%, 14%, 13%, 12%, 11%, 10%, 9%, 8%, 7%, 6%, or 5%.

In some embodiments, the reduction is about 60%, 59%, 58%, 57%, 56%, 55%, 54%, 53%, 52%, 51%, 50%, 49%, 48%, 47%, 46%, 45%, 44%, 43%, 42%, 41%, 40%, 39%, 38%, 37%, 36%, 35%, 34%, 33%, 32%, 31%, 30%, 29%, 28%, 27%, 26%, 25%, 24%, 23%, 22%, 21%, 20%, 19%, 18%, 17%, 16%, 15%, 14%, 13%, 12%, 11%, 10%, 9%, 8%, 7%, 6%, or 5%.

In some embodiments, the reductions in levels of Lp(a) following administration of the pharmaceutical composition as compared to pre-treatment Lp(a) levels is 60%, 59%, 58%, 57%, 56%, 55%, 54%, 53%, 52%, 51%, 50%, 49%, 48%, 47%, 46%, 45%, 44%, 43%, 42%, 41%, 40%, 39%, 38%, 37%, 36%, 35%, 34%, 33%, 32%, 31%, 30%, 29%, 28%, 27%, 26%, 25%, 24%, 23%, 22%, 21%, 20%, 19%, 18%, 17%, 16%, 15%, 14%, 13%, 12%, 11%, 10%, 9%, 8%, 7%, 6%, or 5%.

In some embodiments, the reduction is about 60%, 59%, 58%, 57%, 56%, 55%, 54%, 53%, 52%, 51%, 50%, 49%, 48%, 47%, 46%, 45%, 44%, 43%, 42%, 41%, 40%, 39%, 38%, 37%, 36%, 35%, 34%, 33%, 32%, 31%, 30%, 29%, 28%, 27%, 26%, 25%, 24%, 23%, 22%, 21%, 20%, 19%, 18%, 17%, 16%, 15%, 14%, 13%, 12%, 11%, 10%, 9%, 8%, 7%, 6%, or 5%.

In some embodiments, the reductions in levels of triglycerides (TG) following administration of the pharmaceutical composition as compared to pre-treatment TG levels is 60%, 59%, 58%, 57%, 56%, 55%, 54%, 53%, 52%, 51%, 50%, 49%, 48%, 47%, 46%, 45%, 44%, 43%, 42%, 41%, 40%, 39%, 38%, 37%, 36%, 35%, 34%, 33%, 32%, 31%, 30%, 29%, 28%, 27%, 26%, 25%, 24%, 23%, 22%, 21%, 20%, 19%, 18%, 17%, 16%, 15%, 14%, 13%, 12%, 11%, 10%, 9%, 8%, 7%, 6%, or 5%.

In some embodiments, the reduction is about 60%, 59%, 58%, 57%, 56%, 55%, 54%, 53%, 52%, 51%, 50%, 49%, 48%, 47%, 46%, 45%, 44%, 43%, 42%, 41%, 40%, 39%, 38%, 37%, 36%, 35%, 34%, 33%, 32%, 31%, 30%, 29%, 28%, 27%, 26%, 25%, 24%, 23%, 22%, 21%, 20%, 19%, 18%, 17%, 16%, 15%, 14%, 13%, 12%, 11%, 10%, 9%, 8%, 7%, 6%, or 5%.

In some embodiments, administering the pharmaceutical composition reduces the serum level of LDL-C as compared to LDL-C levels following treatment with placebo. In some embodiments, administering the pharmaceutical composition reduces the serum level of ApoB as compared to ApoB levels following treatment with placebo. In some embodiments, administering the pharmaceutical composition reduces the serum level of non-high density lipoprotein cholesterol (non-HDL-C) as compared to non-HDL-C levels following treatment with placebo. In some embodiments, administering the pharmaceutical composition reduces the serum level of total cholesterol (TC) as compared to TC levels following treatment with placebo. In some embodiments, administering the pharmaceutical composition reduces the serum level of Lp(a) as compared to Lp(a) levels following treatment with placebo. In some embodiments, administering the pharmaceutical composition reduces the serum level of triglycerides (TG) as compared to TG levels following treatment with placebo.

In some embodiments, administering the pharmaceutical composition reduces the fasting serum level of LDL-C as compared to LDL-C levels following treatment with placebo. In some embodiments, administering the pharmaceutical composition reduces the fasting serum level of ApoB as compared to ApoB levels following treatment with placebo. In some embodiments, administering the pharmaceutical composition reduces the fasting serum level of non-high density lipoprotein cholesterol (non-HDL-C) as compared to non-HDL-C levels following treatment with placebo. In some embodiments, administering the pharmaceutical composition reduces the fasting serum level of total cholesterol (TC) as compared to TC levels following treatment with placebo. In some embodiments, administering the pharmaceutical composition reduces the fasting serum level of Lp(a) as compared to Lp(a) levels following treatment with placebo. In some embodiments, administering the pharmaceutical composition reduces the fasting serum level of triglycerides (TG) as compared to TG levels following treatment with placebo.

In some embodiments, the reductions in levels of LDL-C following administration of the pharmaceutical composition compared to LDL-C levels from placebo treatment is 60%, 59%, 58%, 57%, 56%, 55%, 54%, 53%, 52%, 51%, 50%, 49%, 48%, 47%, 46%, 45%, 44%, 43%, 42%, 41%, 40%, 39%, 38%, 37%, 36%, 35%, 34%, 33%, 32%, 31%, 30%, 29%, 28%, 27%, 26%, 25%, 24%, 23%, 22%, 21%, 20%, 19%, 18%, 17%, 16%, 15%, 14%, 13%, 12%, 11%, 10%, 9%, 8%, 7%, 6%, or 5%.

In some embodiments, the reduction is about 60%, 59%, 58%, 57%, 56%, 55%, 54%, 53%, 52%, 51%, 50%, 49%, 48%, 47%, 46%, 45%, 44%, 43%, 42%, 41%, 40%, 39%, 38%, 37%, 36%, 35%, 34%, 33%, 32%, 31%, 30%, 29%, 28%, 27%, 26%, 25%, 24%, 23%, 22%, 21%, 20%, 19%, 18%, 17%, 16%, 15%, 14%, 13%, 12%, 11%, 10%, 9%, 8%, 7%, 6%, or 5%.

In some embodiments, the reductions in levels of ApoB following administration of the pharmaceutical composition compared to ApoB levels from placebo treatment is 60%, 59%, 58%, 57%, 56%, 55%, 54%, 53%, 52%, 51%, 50%, 49%, 48%, 47%, 46%, 45%, 44%, 43%, 42%, 41%, 40%, 39%, 38%, 37%, 36%, 35%, 34%, 33%, 32%, 31%, 30%, 29%, 28%, 27%, 26%, 25%, 24%, 23%, 22%, 21%, 20%, 19%, 18%, 17%, 16%, 15%, 14%, 13%, 12%, 11%, 10%, 9%, 8%, 7%, 6%, or 5%.

In some embodiments, the reduction is about 60%, 59%, 58%, 57%, 56%, 55%, 54%, 53%, 52%, 51%, 50%, 49%, 48%, 47%, 46%, 45%, 44%, 43%, 42%, 41%, 40%, 39%, 38%, 37%, 36%, 35%, 34%, 33%, 32%, 31%, 30%, 29%, 28%, 27%, 26%, 25%, 24%, 23%, 22%, 21%, 20%, 19%, 18%, 17%, 16%, 15%, 14%, 13%, 12%, 11%, 10%, 9%, 8%, 7%, 6%, or 5%.

In some embodiments, the reductions in levels of non-HDL-C following administration of the pharmaceutical composition compared to non-HDL-C levels from placebo treatment is 60%, 59%, 58%, 57%, 56%, 55%, 54%, 53%, 52%, 51%, 50%, 49%, 48%, 47%, 46%, 45%, 44%, 43%, 42%, 41%, 40%, 39%, 38%, 37%, 36%, 35%, 34%, 33%, 32%, 31%, 30%, 29%, 28%, 27%, 26%, 25%, 24%, 23%, 22%, 21%, 20%, 19%, 18%, 17%, 16%, 15%, 14%, 13%, 12%, 11%, 10%, 9%, 8%, 7%, 6%, or 5%.

In some embodiments, the reduction is about 60%, 59%, 58%, 57%, 56%, 55%, 54%, 53%, 52%, 51%, 50%, 49%, 48%, 47%, 46%, 45%, 44%, 43%, 42%, 41%, 40%, 39%, 38%, 37%, 36%, 35%, 34%, 33%, 32%, 31%, 30%, 29%, 28%, 27%, 26%, 25%, 24%, 23%, 22%, 21%, 20%, 19%, 18%, 17%, 16%, 15%, 14%, 13%, 12%, 11%, 10%, 9%, 8%, 7%, 6%, or 5%.

In some embodiments, the reductions in levels of total cholesterol (TC) following administration of the pharmaceutical composition compared to TC levels from placebo treatment is 60%, 59%, 58%, 57%, 56%, 55%, 54%, 53%, 52%, 51%, 50%, 49%, 48%, 47%, 46%, 45%, 44%, 43%, 42%, 41%, 40%, 39%, 38%, 37%, 36%, 35%, 34%, 33%, 32%, 31%, 30%, 29%, 28%, 27%, 26%, 25%, 24%, 23%, 22%, 21%, 20%, 19%, 18%, 17%, 16%, 15%, 14%, 13%, 12%, 11%, 10%, 9%, 8%, 7%, 6%, or 5%.

In some embodiments, the reduction is about 60%, 59%, 58%, 57%, 56%, 55%, 54%, 53%, 52%, 51%, 50%, 49%, 48%, 47%, 46%, 45%, 44%, 43%, 42%, 41%, 40%, 39%, 38%, 37%, 36%, 35%, 34%, 33%, 32%, 31%, 30%, 29%, 28%, 27%, 26%, 25%, 24%, 23%, 22%, 21%, 20%, 19%, 18%, 17%, 16%, 15%, 14%, 13%, 12%, 11%, 10%, 9%, 8%, 7%, 6%, or 5%.

In some embodiments, the reductions in levels of Lp(a) following administration of the pharmaceutical composition compared to Lp(a) levels from placebo treatment is 60%, 59%, 58%, 57%, 56%, 55%, 54%, 53%, 52%, 51%, 50%, 49%, 48%, 47%, 46%, 45%, 44%, 43%, 42%, 41%, 40%, 39%, 38%, 37%, 36%, 35%, 34%, 33%, 32%, 31%, 30%, 29%, 28%, 27%, 26%, 25%, 24%, 23%, 22%, 21%, 20%, 19%, 18%, 17%, 16%, 15%, 14%, 13%, 12%, 11%, 10%, 9%, 8%, 7%, 6%, or 5%.

In some embodiments, the reduction is about 60%, 59%, 58%, 57%, 56%, 55%, 54%, 53%, 52%, 51%, 50%, 49%, 48%, 47%, 46%, 45%, 44%, 43%, 42%, 41%, 40%, 39%, 38%, 37%, 36%, 35%, 34%, 33%, 32%, 31%, 30%, 29%, 28%, 27%, 26%, 25%, 24%, 23%, 22%, 21%, 20%, 19%, 18%, 17%, 16%, 15%, 14%, 13%, 12%, 11%, 10%, 9%, 8%, 7%, 6%, or 5%.

In some embodiments, the reductions in levels of triglycerides (TG) following administration of the pharmaceutical composition compared to TG levels from placebo treatment is 60%, 59%, 58%, 57%, 56%, 55%, 54%, 53%, 52%, 51%, 50%, 49%, 48%, 47%, 46%, 45%, 44%, 43%, 42%, 41%, 40%, 39%, 38%, 37%, 36%, 35%, 34%, 33%, 32%, 31%, 30%, 29%, 28%, 27%, 26%, 25%, 24%, 23%, 22%, 21%, 20%, 19%, 18%, 17%, 16%, 15%, 14%, 13%, 12%, 11%, 10%, 9%, 8%, 7%, 6%, or 5%.

In some embodiments, the reduction is about 60%, 59%, 58%, 57%, 56%, 55%, 54%, 53%, 52%, 51%, 50%, 49%, 48%, 47%, 46%, 45%, 44%, 43%, 42%, 41%, 40%, 39%, 38%, 37%, 36%, 35%, 34%, 33%, 32%, 31%, 30%, 29%, 28%, 27%, 26%, 25%, 24%, 23%, 22%, 21%, 20%, 19%, 18%, 17%, 16%, 15%, 14%, 13%, 12%, 11%, 10%, 9%, 8%, 7%, 6%, or 5%.

In some embodiments, administering the pharmaceutical composition increases the serum level of ApoA1 as compared to pre-treatment levels. In some embodiments, administering the pharmaceutical composition increases the serum level of high-density lipoprotein cholesterol (HDL-C) as compared to pre-treatment levels. In some embodiments, administering the pharmaceutical composition increases the serum level of total cholesterol (TC) as compared to pre-treatment levels.

In some embodiments, administering the pharmaceutical composition increases the fasting serum level of ApoA1 as compared to pre-treatment levels. In some embodiments, administering the pharmaceutical composition increases the fasting serum level of HDL-C as compared to pre-treatment levels. In some embodiments, administering the pharmaceutical composition increases the fasting serum level of total cholesterol (TC) as compared to pre-treatment levels.

In some embodiments, the increase in levels of ApoA1 following administration of the pharmaceutical composition as compared to pre-treatment levels is 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, or 60%.

In some embodiments, the increase reduction is about 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, or 60%.

In some embodiments, the increase in levels of HDL-C following administration of the pharmaceutical composition as compared to pre-treatment levels is 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, 100%, 101%, 102%, 103%, 104%, 105%, 106%, 107%, 108%, 109%, 110%, 111%, 112%, 113%, 114%, 115%, 116%, 117%, 118%, 119%, 120%, 121%, 122%, 123%, 124%, 125%, 126%, 127%, 128%, 129%, 130%, 131%, 132%, 133%, 134%, 135%, 136%, 137%, 138%, 139%, 140%, 141%, 142%, 143%, 144%, 145%, 146%, 147%, 148%, 149%, 150%, 151%, 152%, 153%, 154%, 155%, 156%, 157%, 158%, 159%, or 160%.

In some embodiments, the increase is about 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, 100%, 101%, 102%, 103%, 104%, 105%, 106%, 107%, 108%, 109%, 110%, 111%, 112%, 113%, 114%, 115%, 116%, 117%, 118%, 119%, 120%, 121%, 122%, 123%, 124%, 125%, 126%, 127%, 128%, 129%, 130%, 131%, 132%, 133%, 134%, 135%, 136%, 137%, 138%, 139%, 140%, 141%, 142%, 143%, 144%, 145%, 146%, 147%, 148%, 149%, 150%, 151%, 152%, 153%, 154%, 155%, 156%, 157%, 158%, 159%, or 160%.

In some embodiments, the increase in levels of TC following administration of the pharmaceutical composition as compared to pre-treatment levels is 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, or 60%.

In some embodiments, the reduction is about 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, or 60%.

In some embodiments, administering the pharmaceutical composition increases the serum level of ApoA1 as compared to ApoA1 levels following treatment with placebo. In some embodiments, administering the pharmaceutical composition increases the serum level of high-density lipoprotein cholesterol (HDL-C) as compared to HDL-C levels following treatment with placebo. In some embodiments, administering the pharmaceutical composition increases the serum level of total cholesterol (TC) as compared to TC levels following treatment with placebo.

In some embodiments, administering the pharmaceutical composition increases the fasting serum level of ApoA1 as compared to ApoA1 levels following treatment with placebo. In some embodiments, administering the pharmaceutical composition increases the fasting serum level of high-density lipoprotein cholesterol (HDL-C) as compared to HDL-C levels following treatment with placebo. In some embodiments, administering the pharmaceutical composition increases the fasting serum level of total cholesterol (TC) as compared to TC levels following treatment with placebo.

In some embodiments, the increase in levels of ApoA1 following administration of the pharmaceutical composition compared to ApoA1 levels from placebo treatment is 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, or 60%.

In some embodiments, the increase is about 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, or 60%.

In some embodiments, the increase in levels of HDL-C following administration of the pharmaceutical composition compared to HDL-C levels from placebo treatment is 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, 100%, 101%, 102%, 103%, 104%, 105%, 106%, 107%, 108%, 109%, 110%, 111%, 112%, 113%, 114%, 115%, 116%, 117%, 118%, 119%, 120%, 121%, 122%, 123%, 124%, 125%, 126%, 127%, 128%, 129%, 130%, 131%, 132%, 133%, 134%, 135%, 136%, 137%, 138%, 139%, 140%, 141%, 142%, 143%, 144%, 145%, 146%, 147%, 148%, 149%, 150%, 151%, 152%, 153%, 154%, 155%, 156%, 157%, 158%, 159%, or 160%.

In some embodiments, the increase is about 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, 100%, 101%, 102%, 103%, 104%, 105%, 106%, 107%, 108%, 109%, 110%, 111%, 112%, 113%, 114%, 115%, 116%, 117%, 118%, 119%, 120%, 121%, 122%, 123%, 124%, 125%, 126%, 127%, 128%, 129%, 130%, 131%, 132%, 133%, 134%, 135%, 136%, 137%, 138%, 139%, 140%, 141%, 142%, 143%, 144%, 145%, 146%, 147%, 148%, 149%, 150%, 151%, 152%, 153%, 154%, 155%, 156%, 157%, 158%, 159%, or 160%.

In some embodiments, the increase in levels of total cholesterol (TC) following administration of the pharmaceutical composition compared to TC levels from placebo treatment is 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, or 60%.

In some embodiments, the increase is about 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, or 60%.

In some embodiments, obicetrapib or a pharmaceutically acceptable salt, hydrate, or solvate thereof, is administered orally at a dose of 10 mg once daily. In some embodiments, obicetrapib, or a pharmaceutically acceptable salt thereof, is administered orally at a dose of 5 mg once daily.

### 5.3.Pharmaceutical Compositions

Pharmaceutical compositions disclosed herein comprise, as the active pharmaceutical ingredient (API), obicetrapib or a pharmaceutically acceptable salt, hydrate, or solvate thereof. Obicetrapib is the INN of the compound having the IUPAC name (2R,4S)-4-{[3,5-bis(trifluoromethyl)benzyl]-[5-(3-carboxypropoxy)pyrimidin-2-yl]amino}-2-ethyl-6-trifluoromethyl-3,4-dihydro-2H-quinoline-1-carboxylic acid ethyl ester and the following structural formula:

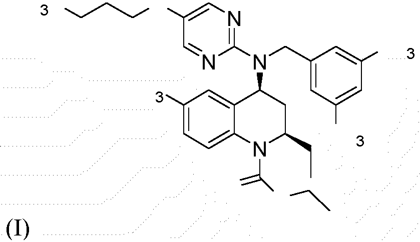

The term "pharmaceutically acceptable" as used herein has its conventional meaning and refers to compounds, material, compositions and/or dosage forms, which are, within the scope of sound medical judgment, suitable for contact with the tissues of mammals, especially humans, without excessive toxicity, irritation, allergic response and other problem complications, commensurate with a reasonable benefit/risk ratio.

As used herein, "a pharmaceutically acceptable salt" includes any salt that retains the activity of the active agent(s) and is acceptable for pharmaceutical use. Pharmaceutically acceptable salts of the compound (I) may include, for example, alkali metal salts such as lithium, sodium or potassium salt; alkali earth metal salts such as calcium or magnesium salt; salts with zinc or aluminum; salts with organic bases such as ammonium, choline, diethanolamine, lysine, ethylenediamine, tert-butylamine, tert-octylamine, tris(hydroxymethyl)aminomethane, N-methylglucosamine, triethanolamine or dehydroabiethylamine; salts with inorganic acids such as hydrochloric acid, hydrobromic acid, hydroiodic acid, sulfuric acid, nitric acid or phosphoric acid; salts with organic acids such as formic acid, acetic acid, propionic acid, oxalic acid, malonic acid, succinic acid, fumaric acid, maleic acid, lactic acid, malic acid, tartaric acid, citric acid, methanesulfonic acid, ethanesulfonic acid, benzenesulfonic acid or toluenesulfonic acid; or salts derived from acidic amino acids such as aspartic acid or glutamic acid. Additionally, the pharmaceutically acceptable salts of the compound (I) may include, for example, quaternary salts formed between a compound of the formula (I) and an alkyl halide or phenylalkyl halide. In certain embodiments, the pharmaceutically acceptable salt is the calcium salt of obicetrapib.

Pharmaceutically acceptable salts of obicetrapib may be prepared by methods well known to those skilled in the art. Synthetic routes are described in EP1730152 and US 7,872,126, in EP2007728 and US 8,084,611, in EP3180314 and US 10,112,904,

Amorphous obicetrapib hemicalcium and methods for its preparation are described in U.S. Patent No. 12,006,305

Furthermore, the compositions can comprise obicetrapib in the form of a solvate, comprising a pharmaceutically acceptable solvent, such as water ('hydrate'), ethanol, and the like. In general, the solvated forms are considered equivalent to the unsolvated forms.

As used herein, the term "pharmaceutical composition" refers to a composition comprising obicetrapib or a salt or solvate thereof and, as the case may be, one or more additional non-toxic ingredients, which composition is in a form suitable for administration to a (human) subject, through any route of administration, and which composition is physiologically tolerated upon such administration.

In a preferred embodiment, the composition comprises one or more carriers and/or excipients. As is known by those of average skill in the art, the appropriate choice of excipients is dependent on multiple factors, including the physicochemical properties of the API, the preferred pharmaceutical form, the preferred route of administration, the desired rate of release, etc. The compositions can be formulated for a variety of routes of administration, oral administration being particularly preferred. It is within the purview of those of average skill in the art to conceive and develop suitable formulations, relying on the common general knowledge as reflected in text books such as Remington: The Science and Practice of Pharmacy (23rd edition, 2020; ISBN: 9780128200070), and routine development efforts.

### 5.4.Further Embodiments

In some embodiments, the presence of HeFH is determined by genotyping.

In some embodiments, wherein HeFH is determined by clinical assessment.

In some embodiments, the presence of ASCVD is determined by the presence of at least one of the conditions selected from the group consisting of: coronary artery disease, cerebrovascular disease, and peripheral arterial disease.

In some embodiments, the subject, prior to first administration of obicetrapib, is on maximally tolerated lipid-modifying therapy.

In some embodiments, the maximally tolerated lipid-modifying therapy comprises at least one treatment selected from the group consisting of: a statin at a maximally tolerated stable dose, ezetimibe, bempedoic acid, a proprotein convertase subtilisin/kexin type 9 (PCSK9)-targeted therapy, a fibrate, provided that the fibrate is not gemfibrozil, and combinations thereof.

In some embodiments, the maximally tolerated lipid-modifying therapy comprises at least one treatment selected from the group consisting of: a statin at a maximally tolerated stable dose, ezetimibe, bempedoic acid, a proprotein convertase subtilisin/kexin type 9 (PCSK9)-targeted therapy, a fibrate, provided that the fibrate is not gemfibrozil, and combinations thereof.

In some embodiments, the subject, prior to first administration of obicetrapib, is on high-intensity statin therapy (HIS).

In some embodiments, the high-intensity statin therapy (HIS) is atorvastatin at 40 mg or 80 mg.

In some embodiments, the high-intensity statin therapy (HIS) is rosuvastatin at 20 mg or 40 mg.

In some embodiments, the subject, prior to first administration of obicetrapib, has a fasting serum LDL-C equal to or greater than 70 mg/dL (1.81 mmol/L).

In some embodiments, the subject, prior to first administration of obicetrapib, has a fasting serum TG less than 400 mg/dL (4.52 mmol/L).

In some embodiments, the subject, prior to first administration of obicetrapib, has an estimated glomerular filtration rate (eGFR) equal to or greater than 30 mL/min/1.73 m2 calculated using the Chronic Kidney Disease Epidemiology Collaboration equation.

In some embodiments, administering the pharmaceutical composition reduces the serum level of LDL-C as compared to pre-treatment levels.

In some embodiments, administering the pharmaceutical composition reduces the serum level of ApoB as compared to pre-treatment levels.

In some embodiments, administering the pharmaceutical composition reduces the serum level of non-HDL-C as compared to pre-treatment levels.

In some embodiments, administering the pharmaceutical composition increases the serum level of total cholesterol (TC) as compared to pre-treatment levels.

In some embodiments, administering the pharmaceutical composition reduces the serum level of Lp(a) as compared to pre-treatment levels.

In some embodiments, administering the pharmaceutical composition reduces the serum level of total triglycerides (TG) as compared to pre-treatment levels.

In some embodiments, administering the pharmaceutical composition increases the serum level of HDL-C as compared to pre-treatment levels.

In some embodiments, administering the pharmaceutical composition increases the serum level of ApoA1 as compared to pre-treatment levels.

In some embodiments, administering the pharmaceutical composition reduces the HbA1c level as compared to pre-treatment levels.

In some embodiments, administering the pharmaceutical composition reduces the HOMA-IR level as compared to pre-treatment levels.

In some embodiments, administering the pharmaceutical composition reduces the blood glucose level as compared to pre-treatment levels.

In some embodiments, administering the pharmaceutical composition reduces the risk of the first occurrence of any event selected from the group consisting of: cardiovascular (CV) death, non-fatal myocardial infarction (MI), non-fatal stroke, and non-elective coronary revascularization.

In some embodiments, administering the pharmaceutical composition reduces the risk of the first occurrence of any event selected from the group consisting of: cardiovascular (CV) or coronary heart disease (CHD) death, non-fatal myocardial infarction (MI), and non-fatal stroke.

In some embodiments, administering the pharmaceutical composition reduces the risk of cardiovascular (CV) or coronary heart disease (CHD) death.

In some embodiments, administering the pharmaceutical composition reduces the risk of non-fatal myocardial infarction (MI).

In some embodiments, administering the pharmaceutical composition reduces the risk of non-fatal stroke.

In some embodiments, administering the pharmaceutical composition reduces the risk of non-elective coronary revascularization.

In some embodiments, administering the pharmaceutical composition reduces the risk of the first occurrence of any event selected from the group consisting of: hospitalization for unstable angina or chest pain, hospitalization for heart failure (HF), and transient ischemic attack (TIA).

In some embodiments, administering the pharmaceutical composition reduces the risk of hospitalization for unstable angina or chest pain.

In some embodiments, administering the pharmaceutical composition reduces the risk of hospitalization for heart failure (HF).

In some embodiments, administering the pharmaceutical composition reduces the risk of transient ischemic attack (TIA).

In some embodiments, obicetrapib or a pharmaceutically acceptable salt, hydrate, or solvate thereof, is administered orally at a dose of 10 mg once daily.

In some embodiments, obicetrapib or a pharmaceutically acceptable salt, hydrate, or solvate thereof is administered daily for at least 6 months.

In some embodiments, obicetrapib or a pharmaceutically acceptable salt thereof is administered for at least 9 months.

In some embodiments, obicetrapib or a pharmaceutically acceptable salt thereof is administered for at least 12 months.

### 6. EXAMPLES

The following examples are provided by way of exemplification and illustration, not limitation.

### 6.1. Example 1: Obicetrapib on Top of Maximum Tolerated Lipid-Modifying Therapies (BROADWAY, NCT05142722): A Placebo-Controlled, Double-Blind, Randomized, Phase 3 Study to Evaluate the Effect of 10 mg Obicetrapib in Participants with Underlying HeFH and/or Atherosclerotic Cardiovascular Disease (ASCVD) Who are Not Adequately Controlled by Their Lipid-Modifying Therapies

### 6.1.1. Obicetrapib

A Phase 3 clinical study was conducted to evaluate the effect of 10 mg of obicetrapib as an adjunct to diet and maximally tolerated lipid-lowering therapy for the treatment of adults with heterozygous familial hypercholesterolemia (HeFH) and/or a history of atherosclerotic cardiovascular disease (ASCVD) who require additional lowering of low-density lipoprotein cholesterol (LDL-C), see clinical trial NCT05142722 protocol

Obicetrapib is a selective cholesteryl ester transfer protein (CETP) inhibitor. Obicetrapib was administered at 10 mg orally, once daily to subjects in the treatment group.

### 6.1.2. Objectives of the Study

The primary objective of this study was to evaluate the effect of obicetrapib on LDL-C levels in subjects with underlying HeFH and/or ASCVD who were not adequately controlled by their lipid-modifying therapies.

The secondary objectives of this study included evaluating the effect of obicetrapib on apolipoprotein B (ApoB), non-high-density lipoprotein cholesterol (non-HDL-C), high-density lipoprotein cholesterol (HDL-C), total cholesterol (TC), and triglycerides (TG) levels. The secondary objectives also included evaluating the effect of obicetrapib on CV death, non-fatal MI, non-fatal stroke, or non-elective coronary revascularization. The secondary objectives further included evaluating the safety and tolerability profile of obicetrapib in a broadly representative population of adult males and females of all ages, including elderly and very elderly participants, assessed by adverse events (AEs), events of special interest (ESIs), vital signs (including **blood** pressure), ECG measurements, and clinical laboratory values.

The exploratory objectives of this study included evaluating the effect of obicetrapib on the proportion of participants achieving prespecified LDL-C targets. The exploratory objectives also included evaluating the **effect** of obicetrapib on biomarkers, including Lp(a), ApoA1, glycosylated hemoglobin (HbA1c), homeostatic model assessment of insulin resistance (HOMA-IR), and blood glucose. The exploratory objectives further included evaluating the effect of obicetrapib on hospitalization for unstable angina and/or chest pain, hospitalization for heart failure (HF), and transient ischemic attack (TIA).

### 6.1.3. Efficacy Endpoints

The primary efficacy endpoint was the percent change from Baseline to Day 84 in LDL-C in the obicetrapib group **compared** to the placebo group.

The **secondary** efficacy endpoints included the following:
- Percent change from Baseline to Day 84 in ApoB in the obicetrapib group compared to the placebo group;
- Percent change from Baseline to Day 84 in non-HDL-C in the obicetrapib group compared to the placebo group;
- Percent change from Baseline to Day 84 in HDL-C in the obicetrapib group compared to the placebo group;
- Percent change from Baseline to Day 180 in LDL-C in the obicetrapib group compared to the placebo group;
- Percent change from Baseline to Day 365 in LDL-C in the obicetrapib group compared to the placebo group;
- The time from Randomization until the first confirmed occurrence of a composite of CV death, non-fatal MI, non-fatal stroke, or non-elective coronary revascularization;
- The time from Randomization until the first confirmed occurrence of a composite of CV death, non-fatal MI, or non-fatal stroke;
- Percent change from Baseline to Day 84 in TC in the obicetrapib group compared to the placebo group; and
- Percent change from Baseline to Day 84 in TG in the obicetrapib group compared to the placebo group.

The exploratory efficacy endpoints included the following:
- Proportion of participants at Day 84 who achieve LDL-C <70 mg/dL in the obicetrapib group compared to the placebo group;
- Proportion of participants at Day 84 who achieve LDL-C <55 mg/dL in the obicetrapib group compared to the placebo group;
- Percent change from Baseline to Day 84 in Lp(a) and ApoA1 in the obicetrapib group compared to the placebo group;
- Percent change from Baseline to Day 365 in HbA1c in the obicetrapib group compared to the placebo group;
- Percent change from Baseline to Day 365 in HOMA-IR in the obicetrapib group compared to the placebo group;
- Percent change from Baseline to Day 365 in blood glucose in the obicetrapib group compared to the placebo group;
- Trough levels of obicetrapib from Baseline to Day 365 in the obicetrapib group; and
- The time from Randomization until the first confirmed occurrence of hospitalization for unstable angina and/or chest pain, hospitalization for HF, and TIA.

### 6.1.4. Study Design

This was a multicenter, placebo-controlled, double-blind, randomized, Phase 3 study in approximately 2500 participants with underlying HeFH and/or a history of ASCVD who were not adequately controlled by their lipid-modifying therapy. Lipid-modifying therapies comprised maximally tolerated statin therapy, with or without ezetimibe, bempedoic acid or proprotein convertase subtilisin kexin type 9 (PCSK9) inhibitors. The participants were randomized in a 2:1 ratio to Obicetrapib group (one 10 mg obicetrapib tablet once daily) or Placebo group (one placebo tablet once daily). Starting on Day 1, each participant self-administered their assigned study drug once daily until Day 365. Unless otherwise stated, baseline values are the last non-missing measurements taken prior to the participant receiving study drug.

Key inclusion criteria included diagnosis of HeFH and/or history of ASCVD. Diagnosis of HeFH was made by either genotyping (analyzed by a central laboratory) or by clinical assessment using the WHO Criteria/Dutch Lipid Clinical Network Criteria with a score that is >8 points or the Simon Broome Register Diagnostic Criteria with an assessment of "definite or possible HeFH." ASCVD comprised the presence of coronary artery disease (history of myocardial infarction, coronary revascularization, angiographic evidence of a coronary artery stenosis greater than 70% or calcium score greater than 100 Agatston units), cerebrovascular disease (ischemic stroke not due to atrial fibrillation, valvular heart disease or mural thrombus, carotid artery stenosis greater than 70% or carotid revascularization) or peripheral arterial disease (resting ankle-brachial index ≤0.85, peripheral artery revascularization or non-traumatic lower limb amputation). History of ASCVD was defined by at least 1 of the following conditions: coronary artery disease, cerebrovascular disease, and peripheral arterial disease. The subjects were on maximally tolerated lipid-modifying therapy, defined as following: a statin at a maximally tolerated stable dose, ezetimibe for at least 4 weeks, bempedoic acid for at least 4 weeks, a proprotein convertase subtilisin/kexin type 9 (PCSK9)-targeted therapy for at least 3 stable doses, and/or a fibrate at a stable dose for at least 6 weeks (with the exception of gemfibrozil, which was not allowed). A majority of the participants enrolled into this study took high-intensity statin therapy (HIS), which included: Atorvastatin 40 and 80 mg; and Rosuvastatin 20 and 40 mg. In the absence of risk enhancing factors, eligibility required either a fasting serum LDL-C ≥100 mg/dL (≥2.59 mmol/L) or non-HDL-C ≥130 mg/dL (≥3.36 mmol/L). In the presence of at least one cardiovascular risk enhancing factor, eligibility required a fasting serum LDL-C ≥55 mg/dL (≥1.42 mmol/L) to <100 mg/dL (<2.59 mmol/L), or non-HDL-C between 85 and 130 mg/dL. Risk enhancing factors included myocardial infarction within the previous 3 to 12 months, type 2 diabetes, current smoking at time of the trial, age greater than 60 year, high sensitivity C-reactive protein ≥2 mg/L, triglycerides >150 mg/dL, lipoprotein(a) >70 nmol/L or HDL cholesterol <40 mg/dL The subjects also have a fasting TG <400 mg/dL (<4.52 mmol/L) and an estimated glomerular filtration rate (eGFR) ≥30 mL/min/1.73 m² calculated using the Chronic Kidney Disease Epidemiology Collaboration equation.

Key exclusion criteria included current or previous history of New York Heart Association class III or IV HF or left ventricular ejection fraction <30%, hospitalization for HF within 5 years prior to Screening, an clinical event including non-fatal myocardial infarction (MI), non-fatal stroke, non-elective coronary revascularization, and/or hospitalization for unstable angina and/or chest pain within 3 months prior to Screening, uncontrolled severe hypertension, defined as either systolic blood pressure ≥160 mmHg or diastolic blood pressure ≥100 mmHg, formal diagnosis of homozygous familial hypercholesterolemia (FH), active liver disease, HbA1c ≥10%, thyroid-stimulating hormone >1.5 × ULN, and creatine kinase (CK) >3 × ULN.

### 6.1.5. Summary Results

Obicetrapib at 10 mg orally once daily significantly reduced LDL-C level in subjects with HeFH and/or ASCVD compared with placebo. Obicetrapib at 10 mg also significantly reduced ApoB, non-HDL-C, and Lp(a) levels compared to placebo. In addition, obicetrapib at 10 mg significantly increased HDL-C and ApoA1 levels compared to placebo.

### 6.1.6. Obicetrapib Lowers LDL-C in HeFH and/or ASCVD Versus Placebo

The primary endpoint was percent change from baseline in LDL-C of obicetrapib 10 mg compared to placebo after 84 days (Day 84). The results observed in the intention-to-treat population (ITT) showed least squares mean reduction in LDL-C from baseline by preparative ultracentrifugation (PUC) with imputation that was 33% greater in the obicetrapib group than in the placebo group (placebo-adjusted reduction) at Day 84 and 24% greater reduction from baseline in the obicetrapib group as compared to the placebo group (placebo-adjusted baseline) at Day 365 *(See* Table 1 and FIG. 1A). (p<0.0001). Results for mean LDL-C reduction from baseline over time culminating at Day 365 are shown in FIG. 1B for the placebo group, obicetrapib 10 mg intention-to-treat (ITT) group, obicetrapib 10 mg on-treatment group, and obicetrapib 10 mg off treatment population.

| **Table 1: LDL-C percentage change at day 84** | | | |
|---|---|---|---|
| | **Placebo (n = 844)** | **Obicetrapib 10 mg (n = 1686)** | **Difference** |
| **Mean** | -1.6% | -34.7% | **-33.1%** |
| **Median** | -4.3% | -40.3% | **-36.0%** |
| **LS mean (with imputation)** | +2.7% | -29.9% | **-32.7%** |

FIG. 1C shows the mean percent change by PUC from baseline over time for placebo and obicetrapib populations (Table 2). Similar results for LDL-C change calculated by PUC were observed for LDL-C change determined by Friedewald and Martin/Hopkins equations (Table 2).

FIG. 1D shows that a lower absolute LDL-C level was achieved with obicetrapib compared to placebo (62.8±37.3 vs 92.3±35.1 mg/dL, P<0.0001). Additionally, a greater percentage of subjects receiving obicetrapib versus placebo achieved LDL cholesterol levels less than 40 mg/dL (27.9 vs 1.1%, P<0.0001), 55 mg/dL (51.0 vs 8.0%, P<0.0001) and 70 mg/dL (68.4 vs 27.5%, P<0.0001) (FIG. 1E). A placebo-adjusted reduction in LDL cholesterol of 24.0% (95% CI -20.1% to -27.9%, P<0.0001) was observed from baseline to day 365 in obicetrapib- receiving subjects.

FIG. 2 displays LDL-C reduction versus baseline for 10 mg obicetrapib at Day 84. It shows that 10 mg obicetrapib per day is effective by Day 84 to decrease LDL-C more than 50% in 33% of subjects with HeFH and/or ASCVD on maximally tolerated LDL-C lowering therapies. Note that for this waterfall analysis, each line of the plot represents a single subject and that 5 subjects increased more than 100%.

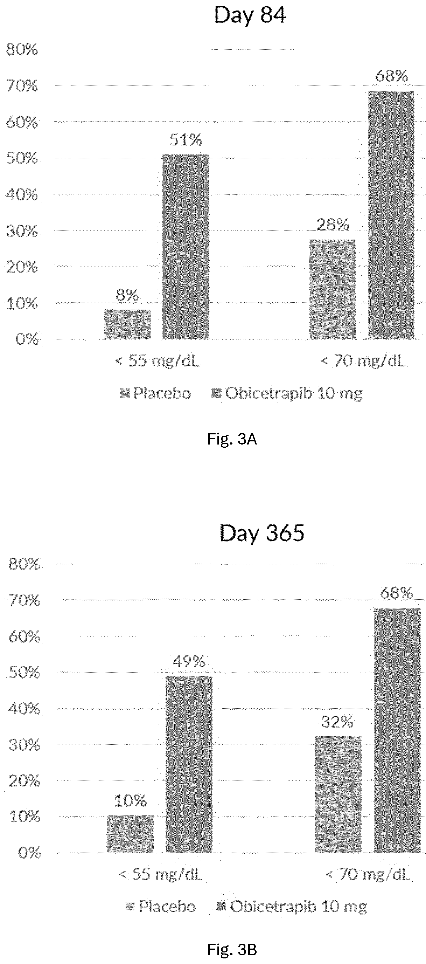

Additionally, the results showed that approximately half of subjects on obicetrapib 10 mg achieved an LDL-C level < 70 mg/dL by Day 84 and an LDL-C level of <55 mg/dL by day 365. *See* FIGS. 3A and 3B.

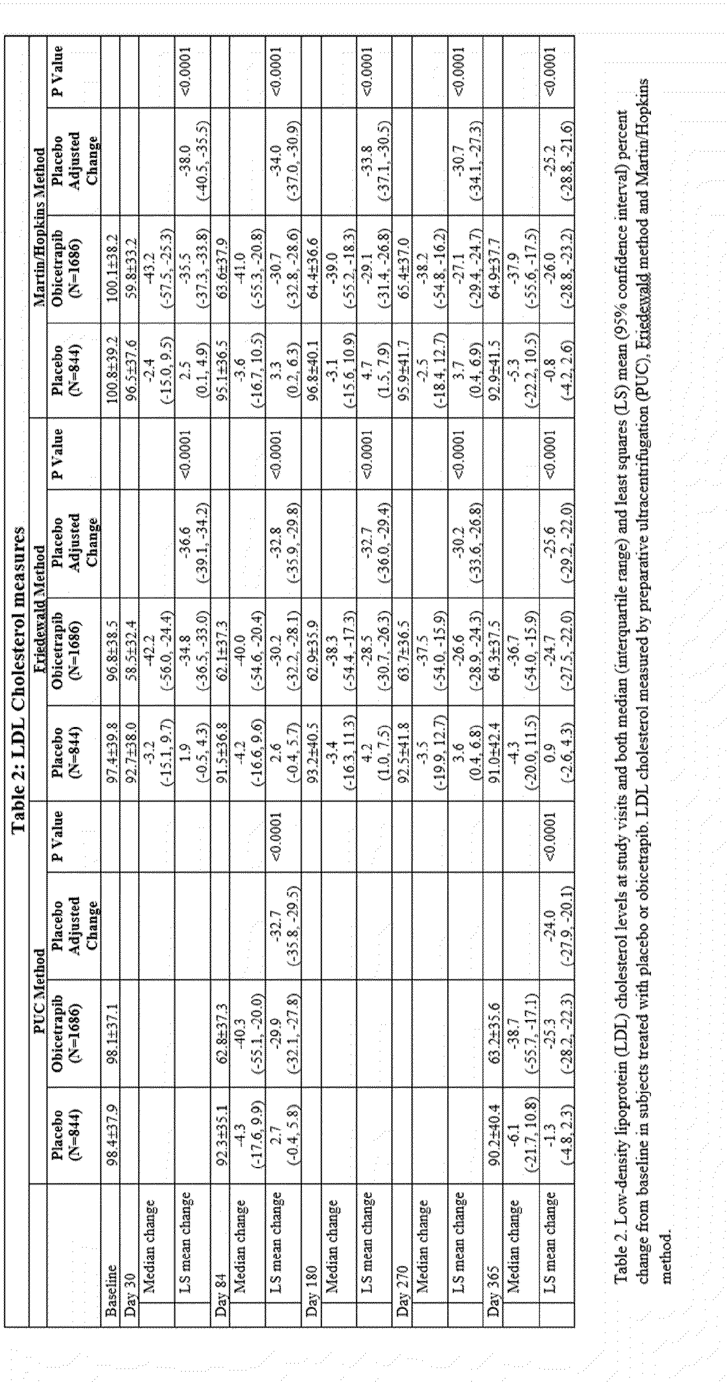

### 6.1.7. Obicetrapib Induces Changes in Other Lipid and Lipoprotein Parameters.

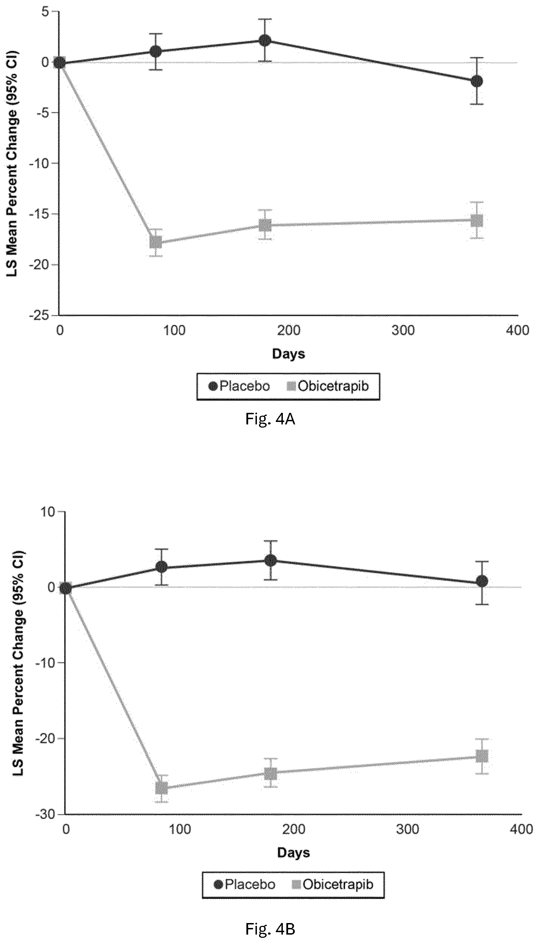

Favorable changes in other lipid and lipoprotein parameters were observed in subjects receiving 10 mg obicetrapib versus placebo. Table 3 and FIGS. 4A-4D summarize the placebo-adjusted changes observed.

### ApoB

Subjects receiving obicetrapib demonstrated percent reductions in apoB at day 84 of -18.9% (95% CI -17.1% to -20.8%, p<0.0001), day 180 of -18.3% (95% CI - 16.2% to -20.4%, p<0.0001) and day 365 of -13.8% (95% CI -11.4% to -16.2% p<0.0001). Table 3 and FIG. 4A.

### Non-HDL Cholesterol

Subjects receiving obicetrapib demonstrated percent reductions in non-HDL-C at day 84 of -29.4% (95% CI -27.0% to -31.9%, P<0.0001), day 180 of -28.3% (95% CI -25.7% to -30.9%, p<0.0001) and day 365 of -23.0% (95% CI -20.0% to -26.1%, p<0.0001). Table 3 and FIG. 4B.

### HDL Cholesterol

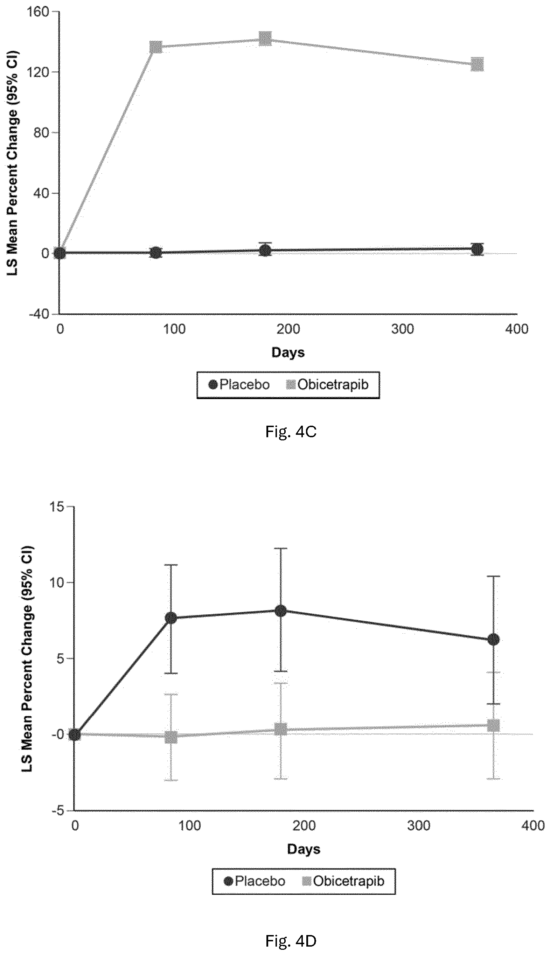

Subjects receiving obicetrapib demonstrated percent increases in HDL cholesterol at day 84 of 136.3% (95% CI 132.5% to 140.1%, p<0.0001), day 180 of 139.9% (95% CI 135.6% to 144.2%, p<0.0001) and day 365 of 122.0% (95% CI 117.5% to 126.6%, p<0.0001). Table 3 and FIG. 4C.

### Triglycerides

Subjects receiving obicetrapib demonstrated percent reductions triglycerides at day 84 of -7.8% (95% CI -4.1% to -11.6%, p<0.0001), day 180 of -8.0% (95% CI -3.8% to -12.1%, p=0.0002) and day 365 by -5.7% (95% CI -1.2% to -10.3%, p=0.01). Table 3 and FIG. 4D.

### Total Cholesterol

Subjects receiving obicetrapib demonstrated percent increases in total cholesterol at day 84 of 17.7% (95% CI 16.1% to 19.3%, P<0.0001), day 180 of 17.8% (95% CI 16.0% to 19.7%, P<0.0001) and day 365 of 18.5% (95% CI 16.4% to 20.7%, P<0.0001). Table 3.

### ApoA1

Subjects receiving obicetrapib demonstrated percent increases in apoA1 at day 84 of 43.2% (95% CI 41.7% to 44.6%, P<0.0001). Table 3.

**Table 3. Least square (LS) mean (95% confidence interval, CI) change in lipid and lipoprotein parameters in subjects treated with placebo or obicetrapib and placebo-adjusted change. HDL, high-density lipoprotein; LDL, low-density lipoprotein.**

| **Table 3: Percent Changes in Lipid and Lipoprotein Parameters** | | | | |
|---|---|---|---|---|
| | **Placebo (N=844)** | **Obicetrapib (N=1686)** | **Placebo Adjusted Changes** | **P Value** |
| **Primary End Point** | | | | |
| LS mean percent change in LDL Cholesterol (95% CI) | | | | |
| Day 84 | 2.7 (-0.4, 5.8) | -29.9 (-32.1, -27.8) | -32.7 (-35.8, -29.5) | <0.0001 |

| **Secondary End Points** | | | | |
|---|---|---|---|---|
| LS mean percent change in LDL Cholesterol (95% CI) | | | | |
| Day 365 | -1.3 (-4.8, 2.3) | -25.3 (-28.2, -22.3) | -24.0 (-27.9, -20.1) | <0.0001 |

| LS mean percent change in apolipoprotein B (95% CI) | | | | |
|---|---|---|---|---|
| Day 84 | 1.1 (-0.7, 2.9) | -17.8 (-19.2, -16.5) | -18.9 (-20.8, -17.1) | <0.0001 |
| Day 180 | 2.2 (0.2, 4.3) | -16.1 (-17.5, -14.6) | -18.3 (-20.4, -16.2) | <0.0001 |
| Day 365 | -1.8 (-4.1, 0.5) | -15.6 (-17.4, -13.8) | -13.8 (-16.2, -11.4) | <0.0001 |

| LS mean percent change in non-HDL Cholesterol (95% CI) | | | | |
|---|---|---|---|---|
| Day 84 | 2.8 (0.4, 5.2) | -26.6 (-28.4, -24.9) | -29.4 (-31.9, -27.0) | <0.0001 |
| Day 180 | 3.7 (1.1, 6.2) | -24.6 (-26.5, -22.7) | -28.3 (-30.9, -25.7) | <0.0001 |
| Day 365 | 0.6 (-2.3, 3.5) | -22.4 (-24.7, -20.1) | -23.0 (-26.1, -20.0) | <0.0001 |

| LS mean percent change in HDL Cholesterol (95% CI) | | | | |
|---|---|---|---|---|
| Day 84 | 0.6 (-2.1, 3.3) | 136.9 (133.2, 140.5) | 136.3 (132.5, 140.1) | <0.0001 |
| Day 180 | 1.9 (-1.1, 5.0) | 141.8 (137.7, 146.0) | 139.9 (135.6, 144.2) | <0.0001 |
| Day 365 | 3.4 (0.1, 6.6) | 125.4 (121.1, 129.7) | 122.0 (117.5, 126.6) | <0.0001 |

| LS mean percent change in Total Cholesterol (95% CI) | | | | |
|---|---|---|---|---|
| Day 84 | 0 (-1.5, 1.5) | 17.7 (16.3, 19.0) | 17.7 (16.1, 19.3) | <0.0001 |
| Day 180 | 0.9 (-0.9, 2.6) | 18.7 (17.2, 20.2) | 17.8 (16.0, 19.7) | <0.0001 |
| Day 365 | -0.6 (-2.6,1.4) | 18.0 (16.2, 19.7) | 18.5 (16.4, 20.7) | <0.0001 |

| LS mean percent change in Triglycerides (95% CI) | | | | |
|---|---|---|---|---|
| Day 84 | 7.7 (4.1, 11.2) | -0.2 (-3.0, 2.7) | -7.8 (-11.6, -4.1) | <0.0001 |
| Day 180 | 8.2 (4.2, 12.3) | 0.3 (-2.9, 3.4) | -8.0 (-12.1, -3.8) | 0.0002 |
| Day 365 | 6.3 (2.1, 10.5) | 0.6 (-2.9, 4.1) | -5.7 (-10.3, -1.2) | 0.01 |

| LS mean percent change in apolipoprotein A1 (95% CI) | | | | |
|---|---|---|---|---|
| Day 84 | 0.3 (-0.9, 1.4) | 43.4 (41.7, 44.6) | 43.2 (41.7, 44.6) | <0.0001 |

### 6.1.8. Obicetrapib Induces MACE Improvement After 6 Months

As part of the safety analysis, this trial adjudicated major adverse cardiovascular events (MACE), including coronary heart disease (CHD) death, non-fatal myocardial infarction, non-fatal stroke, and need for coronary revascularization, in the obicetrapib 10mg dosage compared to placebo, for the entire trial period. See Table 4 below.

**4-point MACE: CV death, non-fatal myocardial infarction, non-fatal stroke, coronary revascularization. MACE was not a primary or secondary endpoint of the BROADWAY trial.**

| **Table 4: Major adverse cardiovascular events at 365 Days** | | | |
|---|---|---|---|
| | Placebo (n = 844) | Obicetrapib 10 mg (n= 1686) | Hazard Ratio |
| All-cause mortality | 12 (1.4%) | 19 (1.1%) | 0.83 |
| CV Death | 6 (0.7%) | 11 (0.7%) | 0.93 |
| First 4-point MACE | 44 (5.2%) | 70 (4.2%) | 0.79 |

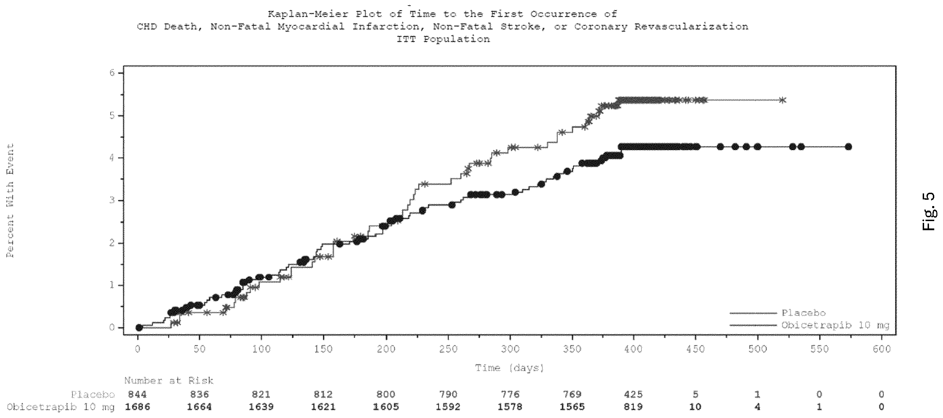

The results of the safety analysis demonstrated a difference in MACE with an early separation in the Kaplan-Meier curves occurring following upon 6 months of daily treatment that were both surprising and unanticipated. *See* FIG. 5. The observed separation after only 6 months' treatment is even more surprising taking into account the separation curves observed in prior trials of statins and other CETP inhibitors: for simvastatin (NCT00202878), see Cannon CP, et al. N Engl J Med 2015;372:2387-2397; for evacetrapib (NCT01687998), see Lincoff AM, et al. N Engl J Med 2017;376:1933-1942; for anacetrapib (NCT01252953), see Bowman L, et al. N Engl J Med 2017;377:1217-1227.

### 6.1.9. Further results regarding baseline lipids (total population mean)

**Table 5: Demographics Indicating Concurrent LDL-C Modifying Therapy**

| | | **Placebo N=884** | **Obicetrapib 10 mg N=1686** |
|---|---|---|---|
| Height (cm) | | 170.1 | 169.9 |
| Weight (kg) | | 86.3 | 85.2 |
| BMI (mean) | | 29.7 | 29.4 |
| | <25 kg/m² | 162 (19.2) | 352 (20.9) |
| | 25 - 30 kg/m² | 334 (39.6) | 657 (39.0) |
| | >30 kg/m² | 346 (41.0) | 676 (40.1) |
| Diabetes n (%) | | 336 (39.8) | 624 (37.0) |
| HeFH | | 143 (16.9) | 284 (16.8) |
| Statin n (%) | | 782 (93.0) | 1533 (91.0) |
| | High Intensity | 581 (68.8) | 1152 (68.3) |
| | Low or Moderate Intensity | 201 (23.8) | 381 (22.6) |
| | None | 62 (7.3) | 153 (9.1) |
| Ezetimibe n (%) | | 220 (26.1) | 453 (26.9) |
| PCSK9i n (%) | | 33 (3.9) | 62 (3.7) |
| | GLP1 n (%) | 48 (5.7) | 111 (6.6) |
| | SGLT2i n (%) | 92 (10.9) | 188 (11.2) |

**Table 6: Demographics**

| | | **Placebo N=884** | **Obicetrapib 10 mg N=1686** |
|---|---|---|---|
| Mean Age (years) | | 65.3 | 65.4 |
| | <65 years | 360 (42.7) | 706 (41.9) |
| | 65 to 74 years | 352 (41.7) | 683 (40.5) |
| | 75+ years | 132 (15.6) | 297 (17.6) |
| Sex (F) n (%) | | 280 (33.2) | 573 (34.0) |
| Region n (%) | | | |
| | North America | 313 (37.1) | 591 (35.1) |
| | Eastern Europe | 282 (33.4) | 580 (34.4) |
| | Western Europe | 104 (12.3) | 213 (12.6) |
| | Asia | 145 (17.2) | 302 (17.9) |
| Race n (%) | | | |
| | White | 647 (76.7) | 1241 (73.6) |
| | Asian | 150 (17.8) | 312 (18.5) |
| | African American | 39 (4.6) | 112 (6.6) |
| | Other | 8 (1.0 | 21 (1.3) |

**Table 7: Disposition of All Randomized Participants**

| | | **Placebo** | **Obicetrapib 10 mg** |
|---|---|---|---|
| Randomized | | 844 | 1686 |
| Completed treatment | | 739 (87.6) | 1499 (88.9) |
| Discontinued Treatment | | 105 (12.4) | 187 (11.1) |
| | Discontinued due to AE's | 43 (5.1) | 68 (4.0) |
| | Subject decision | 33 (3.9) | 57 (3.4) |
| | Lost to follow-up | 12 (1.4) | 30 (1.8) |
| | Withdraw of consent | 6 (0.7) | 7 (0.4) |
| | Death | 6 (0.7) | 8 (0.5) |
| | Other | 5 (0.6) | 17 (1.0) |
| Completed the trial | | 795 (94.2) | 1600 (94.9) |
| Discontinued trial early | | 49 (5.8) | 86 (5.1) |
| Vital status known at trial completion | | 844 (100.0) | 1686 (100.0) |

**Table 8: Overview of Treatment-Emergent Adverse Events at 365 Days - Safety Population**

| | | **Placebo N = 843 n (%)** | **Obicetrapib 10 mg N=1685 n (%)** |
|---|---|---|---|
| Any treatment-emergent AEs (TEAEs) | | 513 (60.9) | 1007 (59.8) |
| Any TEAEs by maximum severity | | | |
| | Mild | 228 (27.0) | 481 (28.5) |
| | Moderate | 217 (25.7) | 408 (24.2) |
| | Severe | 68 (8.1) | 118 (7.0) |
| Any trial drug related TEAEs | | 39 (4.6) | 76 (4.5) |
| Any trial drug-related TEAEs by maximum severity | | | |
| | Mild | 25 (3.0 | 51 (3.0) |
| | Moderate | 14 (1.7) | 23 (1.4) |
| | Severe | 0 (0.0) | 2 (0.1) |
| Any TEAEs leading to discontinuation of trial drug | | 43 (5.1) | 68 (4.0) |
| Any treatment-emergent serious AEs (TESAEs) | | 117 (13.9) | 211 (12.) |
| Any treatment-emergent non-serious Aes | | 495 (58.7) | 959 (56.9) |
| Any trial drug-related TESAEs | | 0 (0.0) | 1 (0.1) |
| Any TEAEs leading to death | | 12 (1.4) | 19 (1.1) |

**Table 9: Overview of Events of Special Interest at 365 Days**

| | | Placebo N = 843 n (%) | Obicetrapib N=1685 n (%) |
|---|---|---|---|
| AST or ALT > 3 x ULN | | 8 (0.9) | 10 (0.6) |
| Bilirubin > 2 x ULN | | 4 (0.5) | 2 (0.1) |
| CK > 5 x ULN | | 3 (0.4) | 5 (0.3) |
| **NODM or worsening of glycemic control** | | 338 (40.1) | 592 (35.1) |
| | - AE indicating new/worse type 1 or 2 diabets | 30 (3.6) | 58 (3.4) |
| | - Initiation of diabetes medication | 104 (12.3) | 186 (11.0) |
| | - HbA1c ≥ 6.5% (where baseline HbA1c <6.5%) | 55 (6.5) | 84 (5.0) |
| | - Two consecutive glucose values > 126 mg/dL | 248 (29.4) | 459 (27.2) |
| | - HbA1c increase from baseline >0.5% | 133 (15.8) | 234 (13.9) |
| | - Worsening glycemic control | 199 (23.6) | 350 (20.8) |
| Renal function worsening | | | |
| | -eGFR <30 mL/min/1.73m2 | 13 (1.5) | 13 (0.8) |
| | - -25% decrease in eGFR from baseline: | 70 (8.3) | 115 (6.8) |
| | - Increase of Serum Creatinine ≥0.3 mg/dL from baseline | 61 (7.2) | 91 (5.4) |
| Macular degeneration | | 0 (0.0) | 1 (0.1) |

**Table 10. Least square (LS) mean (95% confidence interval) change in lipid and lipoprotein parameters in subjects treated with placebo or obicetrapib and placebo-adjusted change. HDL, high-density lipoprotein; LDL, low-density lipoprotein. *Placebo-adjusted percent change in lipoprotein(a) and comparison between the groups analyzed by Hodges-Lehmann estimate given non-parametric distribution at baseline.**

| **Table 10. Lipid and Lipoprotein Parameters.** | | | | | |
|---|---|---|---|---|---|
| | | **Placebo (N=844)** | **Obicetrapib (N=1686)** | **Placebo-Adjusted Changes** | **P Value** |
| **apolipoprotein B** | | | | | |
| Baseline | | 91.9±27.0 | 91.6±26.2 | | |
| Day 84 | | 88.4±25.6 | 70.7±22.0 | | |
| | Median change | -1.5 (-12.9, 8.7) | -23.3 (-35.1, -9.1) | | |
| | LS mean change | 1.1 (-0.7, 2.9) | -17.8 (-19.2, -16.5) | -18.9 (-20.8, -17.1) | <0.0001 |
| Day 180 | | 89.3±27.6 | 71.7±21.2 | | |
| | Median change | -2.6 (-12.8, 9.3) | -21.8 (-34.5, -7.7) | | |
| | LS mean change | 2.2 (0.2, 4.3) | -16.1 (-17.5, -14.6) | -18.3 (-20.4, -16.2) | <0.0001 |
| Day 365 | | 86.3±28.0 | 71.4±22.3 | | |
| | Median change | -3.9 (-17.4, 8.8) | -22.0 (-34.1, -6.9) | | |
| | LS mean change | -1.8 (-4.1, 0.5) | -15.6 (-17.4, -13.8) | -13.8 (-16.2, -11.4) | <0.0001 |

| **non-HDL cholesterol** | | | | | |
|---|---|---|---|---|---|
| Baseline | | 125.6±44.4 | 124.7±43.5 | | |
| Day 84 | | 120.1±42.0 | 84.5±41.2 | | |
| | Median change | -3.2 (-14.8, 9.7) | -35.5 (-47.8, -17.9) | | |
| | LS mean change | 2.8 (0.4, 5.2) | -26.6 (-28.4, -24.9) | -29.4 (-31.9, -27.0) | <0.0001 |
| Day 180 | | 121.4±45.5 | 86.0±39.7 | | |
| | Median change | -2.6 (-14.6, 9.7) | -33.3 (-46.9, -15.6) | | |
| | LS mean change | 3.7 (1.1, 6.2) | -24.6 (-26.5, -22.7) | -28.3 (-30.9, -25.7) | <0.0001 |
| Day 365 | | 118.7±47.7 | 87.2±41.9 | | |
| | Median change | -4.2 (-19.1, 11.5) | -32.8 (-47.9, -14.6) | | |
| | LS mean change | 0.6 (-2.3, 3.5) | -22.4 (-24.7, -20.1) | -23.0 (-26.1, -20.0) | <0.0001 |

| **HDL cholesterol** | | | | | |
|---|---|---|---|---|---|
| Baseline | | 49.7±14.9 | 49.5±14.8 | | |
| Day 84 | | 49.3±15.8 | 113.3±33.4 | | |
| | Median change | 0 (-10.0, 8.9) | 140.8 (100.0, 180.9) | | |
| | LS mean change | 0.6 (-2.1, 3.3) | 136.9 (133.2, 140.5) | 136.3 (132.5, 140.1) | <0.0001 |
| Day 180 | | 50.0±16.0 | 113.8±34.8 | | |
| | Median change | 0 (-9.8, 9.5) | 141.3 (98.8, 184.4) | | |
| | LS mean change | 1.9 (-1.1, 5.0) | 141.8 (137.7, 146.0) | 139.9 (135.6, 144.2) | <0.0001 |
| Day 365 | | 50.7±16.8 | 112.4±36.0 | | |
| | Median change | 0 (-10.2, 13.0) | 140.8 (92.3, 183.3) | | |
| | LS mean change | 3.4 (0.1, 6.6) | 125.4 (121.1, 129.7) | 122.0 (117.5, 126.6) | <0.0001 |

| **Total cholesterol** | | | | | |
|---|---|---|---|---|---|
| Baseline | | 175.3±45.2 | 174.2444.6 | | |
| Day 84 | | 169.4±42.9 | 197.8±49.0 | | |
| | Median change | -2.3 (-11.4, 6.4) | 15.2 (0, 31.1) | | |
| | LS mean change | 0 (-1.5, 1.5) | 17.7 (16.3, 19.0) | 17.7 (16.1, 19.3) | <0.0001 |
| Day 180 | | 171.4±45.9 | 199.8±47.6 | | |
| | Median change | -2.2 (-10.5, 7.7) | 16.3 (0.9, 32.7) | | |
| | LS mean change | 0.9 (-0.9, 2.6) | 18.7 (17.2, 20.2) | 17.8 (16.0, 19.7) | <0.0001 |
| Day 365 | | 169.4±48.4 | 199.6±48.5 | | |
| | Median change | -2.5 (-13.3, 8.6) | 17.8 (0, 33.6) | | |
| | LS mean change | -0.6 (-2.6, 1.4) | 18.0 (16.2, 19.7) | 18.5 (16.4, 20.7) | <0.0001 |

| **Triglycerides** | | | | | |
|---|---|---|---|---|---|
| Baseline | | 127.0 (91.0, 175.0) | 122.0 (91.0, 170.0) | | |
| Day 84 | | 126.0 (91.0, 179.0) | 113.0 (87.0, 150.0) | | |
| | Median change | 0 (-19.7, 24.3) | -6.7 (-24.8, 15.9) | | |
| | LS mean change | 7.7 (4.1, 11.2) | -0.2 (-3.0, 2.7) | -7.8 (-11.6, -4.1) | <0.0001 |
| Day 180 | | 124.5 (90.0, 176.0) | 111.0 (85.0, 150.5) | | |
| | Median change | -1.2 (-21.3, 25.4) | -7.2 (-26.2, 17.2) | | |

| **Table 10. Lipid and Lipoprotein Parameters.** | | | | | |
|---|---|---|---|---|---|
| | | **Placebo (N=844)** | **Obicetrapib (N=1686)** | **Placebo-Adjusted Changes** | **P Value** |
| | LS mean change | 8.2 (4.2, 12.3) | 0.3 (-2.9, 3.4) | -8.0 (-12.1, -3.8) | 0.0002 |
| Day 365 | | 122.0 (88.0, 170.0) | 112.0 (86.0, 154.0) | | |
| | Median change | -2.0 (-21.0, 22.8) | -7.0 (-24.3, 17.4) | | |
| | LS mean change | 6.3 (2.1, 10.5) | 0.6 (-2.9, 4.1) | -5.7 (-10.3, -1.2) | 0.01 |

| **Lipoprotein(a)** | | | | | |
|---|---|---|---|---|---|
| Baseline | | 40.7 (11.8, 159.1) | 39.2 (11.2, 166.3) | | |
| Day 84 | | 38.4 (10.6, 151.3) | 18.0 (4.6, 131.3) | | |
| | Median change | -0.9 (-15.7, 13.0) | -32.3 (-62.8, -4.7) | -33.5 (-36.9, -30.2) | <0.0001* |

| **apolipoprotein A1** | | | | | |
|---|---|---|---|---|---|
| Baseline | | 156.3±26.6 | 155.2±27.7 | | |
| Day 84 | | 154.9±26.7 | 220.2±42.5 | | |
| | Median change | -0.8 (-7.3, 6.6) | 44.1 (28.8, 58.2) | | |
| | LS mean change | 0.3 (-0.9, 1.4) | 43.4 (41.7, 44.6) | 43.2 (41.7, 44.6) | <0.0001 |

In summary, these results show that 10 mg obicetrapib provides durable LDL-C lowering with a surprisingly effective safety and tolerability profile in subjects with HeFH and/or ASCVD and who are not adequately controlled by their lipid-modifying therapy. The placebo-adjusted results also show that 10 mg obicetrapib is capable of decreasing apoB by 18.9%, non-HDL-C by 29.4%, and triglycerides by 7.8%, as well as increasing HDL-C by 136.3% and apoA1 by 43.2%. Additionally, treatment with 10 mg obicetrapib resulted in improvement in glycemic measures that are linked to high CV risk, as well as a difference in major adverse cardiovascular events ("MACE") evident after only 6 months of daily administration of obicetrapib.

These results demonstrate that 10 mg obicetrapib has a positive impact in LDL-C reduction in subjects with elevated LDL-C for whom existing therapies are not sufficiently effective or well tolerated as well as a positive impact on other lipid and lipoprotein parameters associated with ASCVD and ASCVD risk.

### 6.2. Example 2: Impact of Obicetrapib on Cardiovascular Biomarkers and Events: A Pooled Analysis of Phase 3 Clinical Trials

A Phase 3 randomized, double-blind, placebo-controlled clinical study (BROOKLYN, NCT05425745) was conducted to evaluate the effect of 10 mg of obicetrapib as an adjunct to diet and maximally tolerated lipid-lowering therapy for the treatment of adults with heterozygous familial hypercholesterolemia (HeFH) who require additional lowering of low-density lipoprotein cholesterol (LDL-C), see clinicaltrials.gov record NCT05425745 and international application WO 2025/059594

As described in Example 1 (section 6.1) above, a Phase 3 clinical study (BROADWAY, NCT05142722) was conducted to evaluate the effect of 10 mg of obicetrapib daily as an adjunct to diet and maximally tolerated lipid-lowering therapy for the treatment of adults with heterozygous familial hypercholesterolemia (HeFH) and/or a history of atherosclerotic cardiovascular disease (ASCVD) who require additional lowering of low-density lipoprotein cholesterol (LDL-C). The BROADWAY trial and results are discussed in section 6.1 above.

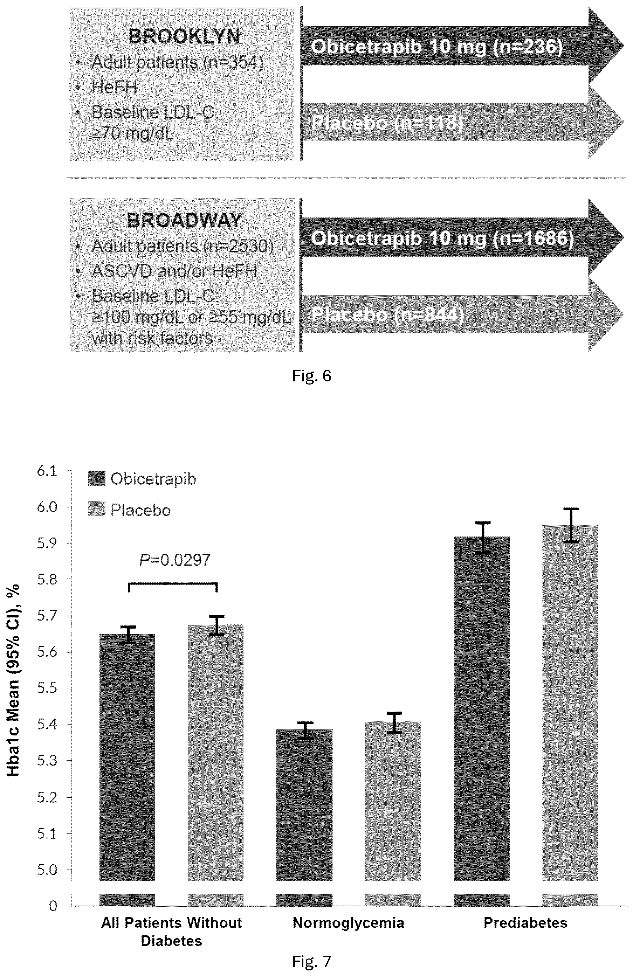

Applicants conducted a pooled analysis of BROOKLYN (NCT05425745) and BROADWAY (NCT05142722) trials to evaluate the effect of 10 mg obicetrapib daily as an adjunct to maximally tolerated lipid-lowering therapy. See FIG. 6.

### 6.2.1. Obicetrapib Lowers Risk of New-Onset Diabetes in Patients with Elevated LDL-C Receiving Maximally Tolerated Statin Therapy

For the 1848 total patients included in the pooled analysis, the mean age was 63.4 years, 38.1% were women, 89.7% were on a statin, median LDL-C was 96 mg/dL (interquartile range [IQR] 78-126), and HbA1c was 5.7% (5.4%-5.9%) at baseline. The baseline clinical characteristics across treatment groups are provided below in Table 11.

| Table 11: Baseline Clinical Characteristics | | |
|---|---|---|
| Parameter, mean (SD) or n (%) | Obicetrapib (n=1249) | Placebo (n=599) |
| Age, years | 63.5 (11.3) | 63.2 (11.1) |
| Female | 478 (38.3) | 226 (37.7) |
| LDL-C, mmol/L | 2.73 (1.08) | 2.78 (1.18) |
| Statin | 1123 (89.9) | 534 (89.1) |
| Prediabetes* | 647 (51.8) | 290 (48.4) |
| Normoglycemia* | 602 (48.2) | 309 (51.6) |
| HbA1c | 5.7 (0.5) | 5.7 (0.4) |

The results showed that placebo-corrected median changes in LDL-C, Lp(a), and HDL-C were -35.3%, -35.6%, and +136.7%, respectively (all *P*<0.0001). (Data not shown).

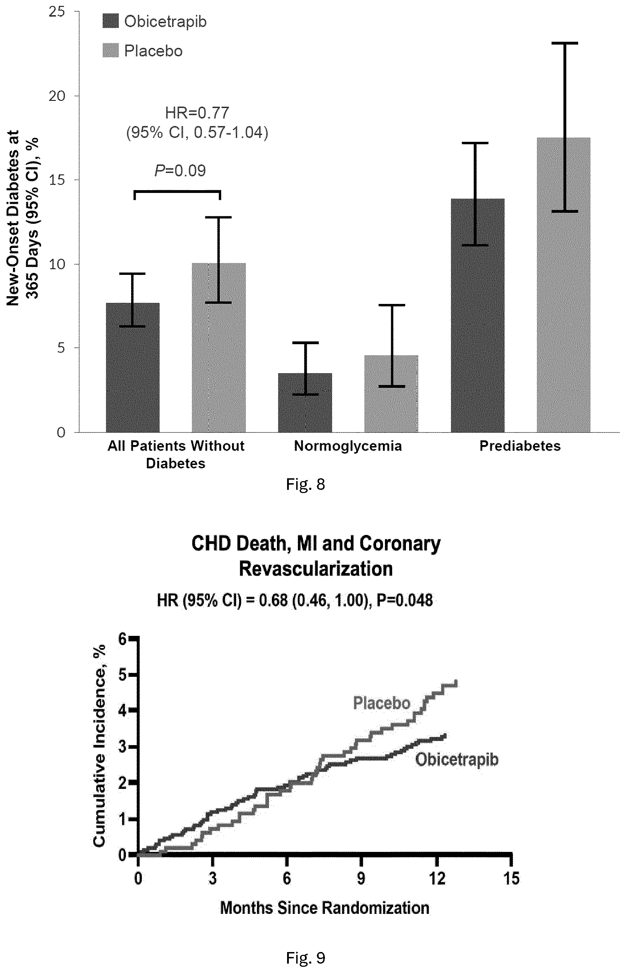

The effect of obicetrapib on hemoglobin A1c (HbA1c) and risk of New-Onset Diabetes was assessed in (a) all subjects without diabetes at baseline, (b) subjects who were normoglycemic at baseline, and (c) subjects who had prediabetes at baseline, with glycemia status determined using standard criteria with adjustments for baseline HbA1c, trial parameters, and statin use at baseline. Results are shown in **FIG. 7****,** and demonstrate that obicetrapib 10 mg significantly reduced hemoglobin A1c (HbA1c) among patients without diabetes and demonstrated a consistent trend toward lower risk of new-onset diabetes (NoD) in patients who were normoglycemia and patients who were prediabetic at baseline. Results shown in **FIG. 8** show a reduction in new-onset diabetes in (a) all subjects without diabetes at baseline, (b) subjects who were normoglycemic at baseline, and (c) subjects who had prediabetes at baseline.

### 6.2.2. Obicetrapib Reduces the Incidence of Major Adverse Cardiovascular Events (MACE)

In a pooled analysis of the data from BROOKLYN and BROADWAY clinical trials, Applicant investigated the effects of obicetrapib on major adverse cardiovascular events (MACE) in patients treated with obicetrapib as an adjunct to maximally tolerated lipid lowering therapy (LLT) compared to placebo.

### Baseline Characteristics

The clinical characteristics and baseline use of lipid lowering medications across treatment groups are summarized in Table 12.

**Table 12. Clinical characteristics and use of lipid lowering medications at baseline in patients treated with placebo or obicetrapib. Continuous parameters presented as median (interquartile range) and categorical parameters as number (percentage). ASCVD, atherosclerotic cardiovascular disease; FH, familial hypercholesterolemia; PCSK9, proprotein convertase subtilisin kexin type 9.**

| Table 12: Clinical characteristics and medication use at baseline | | | | |
|---|---|---|---|---|
| **Parameter** | | **Total (N=2884)** | **Placebo (N=962)** | **Obicetrapib (N=1922)** |
| Age | | 66 (58, 72) | 65 (58, 71) | 66 (58, 72) |
| Females | | 1043 (36.2) | 345 (35.9) | 698 (36.3) |
| White race | | 2217 (76.9) | 757 (78.7) | 1460 (76.0) |
| Body mass index | | 28.8 (25.7, 32.6) | 28.9 (25.8, 32.6) | 28.7 (25.6, 32.5) |
| History of ASCVD | | 2378 (82.5) | 793 (82.4) | 1585 (82.5) |

| **Parameter** | | **Total (N=2884)** | **Placebo (N=962)** | **Obicetrapib (N=1922)** |
|---|---|---|---|---|
| Heterozygous FH | | 777 (26.9) | 260 (27.0) | 517 (26.9) |
| Diabetes | | 1033 (35.8) | 362 (37.6) | 671 (34.9) |
| Hypertension | | 2225 (77.1) | 754 (78.4) | 1471 (76.5) |
| Current smoker | | 599 (20.8) | 197 (20.5) | 402 (20.9) |

| **Medication use** | | | | |
|---|---|---|---|---|
| | Statins | 2623 (91.0) | 881 (91.6) | 1742 (90.6) |
| | High intensity statins | 1999 (69.3) | 661 (68.7) | 1338 (69.6) |
| | Ezetimibe | 859 (29.8) | 279 (29.0) | 580 (30.2) |
| | PCSK9 inhibitor | 153 (5.3) | 59 (6.1) | 94 (4.9) |

Patients had a mean age of 66 years, 36.0% were female, and 77.0% identified as white. Medical history included atherosclerotic cardiovascular disease in 82.0%, heterozygous familial hypercholesterolemia in 27.0% and diabetes in 35.0%. Concomitant use of lipid lowering medications at baseline included statins in 91.0%, high intensity statins in 69.3%, ezetimibe in 29.8% and PCSK9 inhibitors in 5.3%.

### Lipid and Lipoprotein Levels

The lipid and lipoprotein levels at baseline, on treatment, and serial changes in patients treated with placebo or obicetrapib are summarized in Table 13.

**Table 13. Lipid and lipoprotein levels at baseline, 12 months and change in patients treated with placebo or obicetrapib presented as median (interquartile range). *On-treatment lipoprotein(a) was measured at 12 weeks.**

| **Table 13: Lipids and lipoproteins** | | | | |
|---|---|---|---|---|
| **Parameter** | | **Placebo (N=962)** | **Obicetrapib (N=1922)** | **P Value** |
| Low-density lipoprotein cholesterol | | | | |
| | Baseline (mg/dL) | 92.0 (75.0, 122.0) | 93.0 (76.0, 122.0) | 0.82 |
| | 12 months (mg/dL) | 87.0 (68.0, 112.0) | 57.0 (40.0, 85.0) | <0.0001 |
| | Absolute change (mg/dL) | -4.0 (-21.0, 9.0) | -34.0 (-55.0, -14.0) | <0.0001 |
| | Percent change | -4.6 (-20.8, 10.9) | -37.8 (-55.2, -16.8) | <0.0001 |

| High-density lipoprotein cholesterol | | | | |
|---|---|---|---|---|
| | Baseline (mg/dL) | 48.0 (39.0, 57.0) | 48.0 (39.0, 58.0) | 0.73 |
| | 12 months (mg/dL) | 48.0 (40.0, 59.0) | 117.0 (93.0, 136.0) | <0.0001 |
| | Absolute change (mg/dL) | 1.0 (-5.0, 6.0) | 68.0 (48.0, 83.0) | <0.0001 |
| | Percent change | 1.5 (-9.8, 13.6) | 140.0 (92.2, 181.8) | <0.0001 |

| Triglycerides | | | | |
|---|---|---|---|---|
| | Baseline (mg/dL) | 127.0 (91.0, 175.0) | 122.0 (89.0, 170.0) | 0.13 |
| | 12 months (mg/dL) | 122.0 (87.0, 169.5) | 112.0 (85.0, 152.0) | 0.0007 |
| | Absolute change (mg/dL) | -2.0 (-31.0, 23.5) | -8.0 (-36.0, 17.0) | 0.003 |
| | Percent change | -2.3 (-22.6, 22.7) | -6.7 (-24.4, 18.0) | 0.009 |

| Apolipoprotein B | | | | |
|---|---|---|---|---|
| **Table 13: Lipids and lipoproteins** | | | | |
| **Parameter** | | **Placebo (N=962)** | **Obicetrapib (N=1922)** | **P Value** |
| | Baseline (mg/dL) | 88.0 (74.0, 108.0) | 88.0 (75.0, 107.0) | 0.73 |
| | 12 months (mg/dL) | 82.0 (69.0, 101.0) | 68.0 (58.0, 83.0) | <0.0001 |
| | Absolute change (mg/dL) | -3.0 (-15.0, 8.0) | -19.0 (-33.0, -5.0) | <0.0001 |
| | Percent change | -3.6 (-16.9, 9.1) | -21.7 (-34.2, -6.6) | <0.0001 |

| Non-high-density lipoprotein cholesterol | | | | |
|---|---|---|---|---|
| | Baseline (mg/dL) | 116.0 (96.0, 151.0) | 116.0 (97.0, 149.0) | 1.00 |
| | 12 months (mg/dL) | 111.0 (90.0, 143.0) | 79.0 (59.0, 109.0) | <0.0001 |
| | Absolute change (mg/dL) | -4.0 (-22.5, 13.0) | -36.0 (-60.0, -15.0) | <0.0001 |
| | Percent change | -3.7 (-18.4, 11.6) | -32.4 (-47.7, -13.9) | <0.0001 |

| Lipoprotein(a)* | | | | |
|---|---|---|---|---|
| | Baseline (nmol/L) | 40.0 (12.0, 163.8) | 40.5 (11.3, 167.0) | 0.91 |
| | 12 weeks (nmol/L) | 38.4 (10.9, 156.2) | 18.2 (4.6, 129.8) | <0.0001 |
| | Absolute change (nmol/L) | 0 (-7.5, 5.0) | -9.8 (-33.7, -1.5) | <0.0001 |
| | Percent change | 0.0 (-15.2, 13.6) | -32.5 (-63.0, -5.1) | <0.0001 |

At baseline, patients had median levels of LDL-C of 92.0 mg/dL, HDL-C of 48.0 mg/dL, triglycerides of 127.0 mg/dL, apoB of 88.0 mg/dL, non-HDL-C of 116.0 mg/dL and Lp(a) of 40.0 nmol/L. The results show that compared with the placebo group, patients treated with obicetrapib demonstrated greater absolute and percentage reductions in LDL-C (-34.0 vs -4.0 mg/dL; -37.8% vs -4.6%, P<0.0001), apoB (-19.0 vs -3.0 mg/dL; -21.7% vs -3.6%, P<0.0001), non-HDL-C (-36.0 vs -4.0 mg/dL; -32.4% vs -3.7%, P<0.0001) and Lp(a) (-9.8 vs 0 nmol/L; -32.5% vs 0%, P<0.0001) and a greater increase in HDL-C (+68.0 vs _1.0 mg/dL; +140.0% vs +1.5%, P<0.0001).

### Cardiovascular Outcomes

The incidence of cardiovascular events in patients treated with placebo or obicetrapib is summarized in Table 14.

**Table 14. Percent incidence of 4-component (top) and 3-component (bottom) major adverse cardiovascular events (MACE) in patients treated with placebo or obicetrapib for the entire duration of the pooled studies and stratified according to time from randomization to study drug. Hazard ratios (HR), confidence intervals (CI), and P values reflect stratification by study, presence or absence of heterozygous familial hypercholesterolemia, and intensity of baseline statin use, using randomization stratification factors for BROADWAY and derived for BROOKLYN.**

| **Table 14: Cardiovascular events** | | | | | |
|---|---|---|---|---|---|
| **4 Component MACE: CHD death, nonfatal MI, ischemic stroke and coronary revascularization** | | | | | |
| | **Placebo (N=962)** | **Obicetrapib (N=1922)** | **HR (95% CI)** | **HR (95% CI) per 1 mmol/L difference in LDL-C** | **P Value** |
| Total | 5.0 | 3.9 | 0.77 (0.54, 1.11) | 0.72 (0.45, 1.14) | 0.16 |
| | | | | | |

| **Time since randomization** | **HR (95% CI)** | **Wald P Value** | **P Value for Comparison against Constant HR** | | |
|---|---|---|---|---|---|
| ≤6 months | 1.03 (0.59, 1.78) | 0.92 | 0.15 | | |
| >6 months | 0.60 (0.37, 0.99) | 0.04 | | | |
| **3 Component MACE: CHD death, nonfatal MI and coronary revascularization** | | | | | |

| | **Placebo (N=962)** | **Obicetrapib (N=1922)** | **HR (95% CI)** | **HR (95% CI) per 1 mmol/L difference in LDL-C** | **P Value** |
|---|---|---|---|---|---|
| Total | | | | **0.61 (0.37, 1.00)** | |
| | **4.7** | **3.2** | **0.68 (0.46, 1.00)** | | **0.048** |
| | | | | | |

| **Time since randomization** | **HR (95% CI)** | **Wald P Value** | **P Value for Comparison against Constant HR** | | |
|---|---|---|---|---|---|
| ≤6 months | 1.06 (0.60, 1.90) | 0.83 | 0.03 | | |
| >6 months | 0.45 (0.26, 0.77) | 0.003 | | | |

A total of 123 patients (4.3%) experienced at least one cardiovascular event during the 12-month period (coronary heart disease death 0.4%, nonfatal myocardial infarction 1.1%, ischemic stroke 0.7%, coronary revascularization 2.1%).

The data show that the composite of coronary heart disease death, nonfatal myocardial infarction, ischemic stroke and coronary revascularization (4-component MACE) showed a reduced incidence of 3.9% in patients treated with obicetrapib compared to 5.0% in patients treated with placebo. **FIG. 9****.**

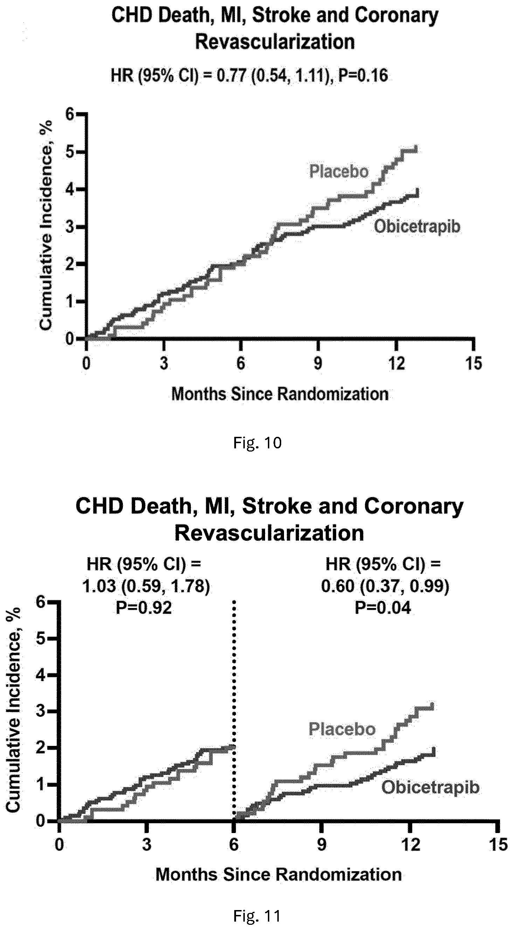

Similarly, the composite of coronary heart disease death, nonfatal myocardial infarction and coronary revascularization (3-component MACE) showed reduced incidence in patients treated with obicetrapib (3.2%) compared to placebo (4.7%). **FIG. 10****.**

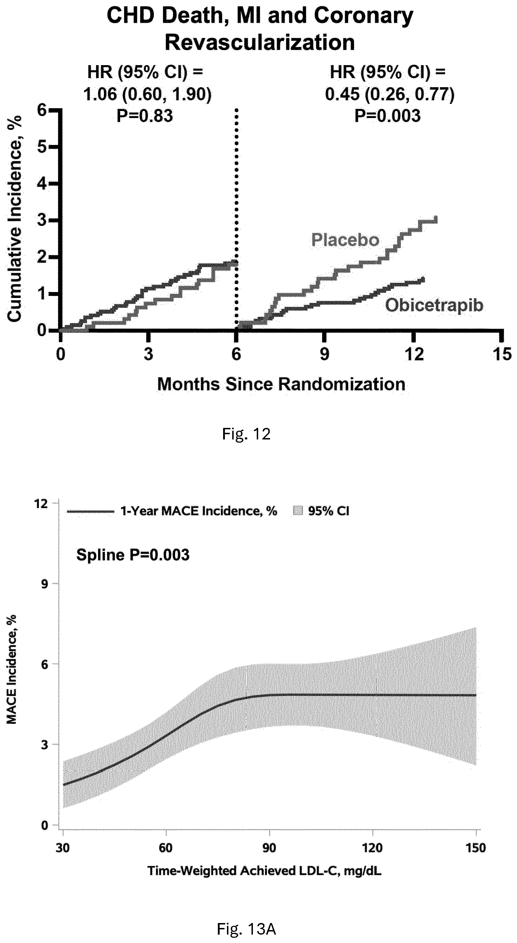

Testing of the interaction between treatment and logarithm follow-up time suggested violation of the proportional hazards for both 4-component (p=0.04) and 3-component (p=0.007) MACE. The cumulative incidence curves indicated minimal treatment differences through 6 months after randomization and risk reduction by obicetrapib more than 6 months after randomization. When allowing the hazard ratio (HR) to have different values before and after this timepoint, a reduction in 4-component MACE with obicetrapib was observed in patients during the period more than 6 months after randomization (HR 95% CI, 0.60, 0.37-0.99, P=0.04) **(****FIG. 11****,** right panel), but not during the first 6 months following randomization (HR 95% CI, 1.03, 0.59-1.78, P=0.92; P=0.15 for comparison against the model assuming a constant treatment effect) **(****FIG. 11****,** left panel). Similarly, a reduction in 3-component MACE with obicetrapib was observed in patients during the period more than 6 months after randomization (HR 95% CI, 0.45, 0.26-0.77, P=0.003) **(****FIG. 12****,** right panel), but not during the first 6 months following randomization (HR 95% CI, 1.06, 0.60-1.90, P=0.83; P=0.03 for comparison against the model assuming a constant treatment effect) **(****FIG. 12****,** left panel).

### Lipids, Lipoproteins and Cardiovascular Outcomes

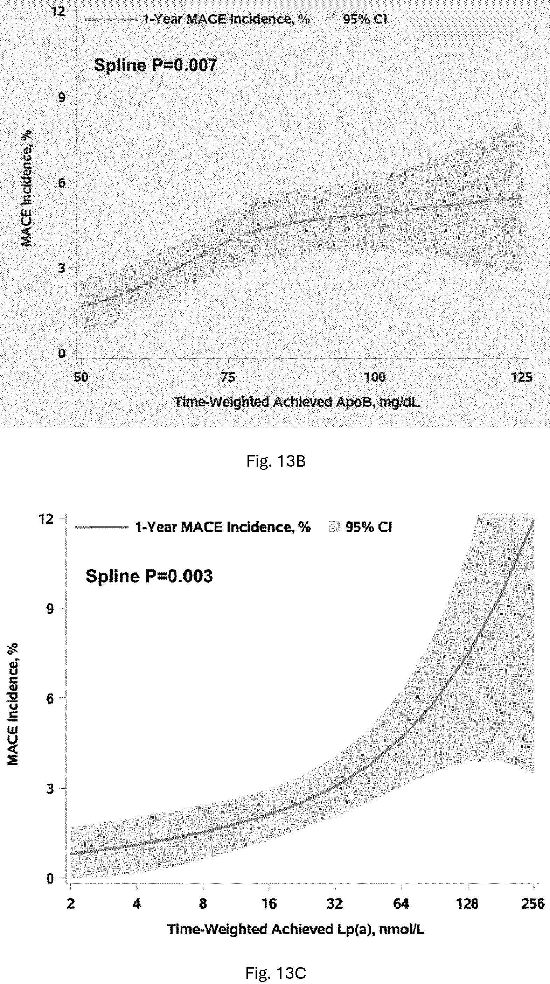

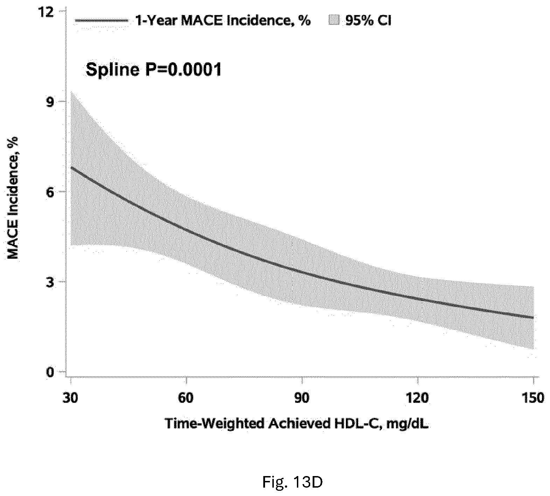

Applicant analyzed the association between time-weighted achieved levels of various lipid biomarkers and the 12-month incidence of 4-component MACE (95% CI). The results showed that the time-weighted LDL-C, HDL-C, and Lp(a) during follow-up was 90.1 (74.1, 116.6) mg/dL, 48.1 (39.5, 58.7) mg/dL, and 39.8 (12.2, 163.0) nmol/L in the placebo group and 61.0 (44.9, 85.7) mg/dL, 111.5 (90.3, 128.3) mg/dL, and 29.1 (8.2, 148.4) nmol/L in the obicetrapib group. The association between time-weighted achieved levels of lipid and lipoprotein parameters and the 1-year incidence of cardiovascular death, nonfatal myocardial infarction, ischemic stroke and coronary revascularization are illustrated in **FIG. 13A-13D****.** Spline analysis, adjusting for the presence of HeFH, use of high intensity statins, study and baseline lipid or lipoprotein level demonstrated a direct association between the incidence of cardiovascular events and achieved levels of both LDL-C (p=0.003), apoB (p=0.007), non-HDL-C (p=0.01) and Lp(a) (p=0.003), and an inverse association with achieved levels of HDL-C (p=0.0001).

### Summary

This pooled analysis of two phase 3 clinical trials, BROOKLYN and BROADWAY, evaluating the impact of treatment with obicetrapib in patients on maximally tolerated lipid lowering therapies and at high cardiovascular risk showed that compared to placebo, obicetrapib produced greater reductions in LDL-C (-34.0 vs -4.0 mg/dL; -37.8% vs -4.6%), apoB (-19.0 vs -3.0 mg/dL; - 21.7% vs -3.6%), non-HDL-C (-36.0 vs -4.0 mg/dL; -32.4% vs -3.7%) and Lp(a) (-9.8 vs 0 nmol/L; -32.5% vs 0%) and increased HDL-C (+68.0 vs +1.0 mg/dL; +140.0% vs +1.5%).

The data also demonstrated that obicetrapib reduced the incidence of major adverse cardiovascular events during 12 months of follow up. 4-component major adverse cardiovascular events (MACE) for coronary heart disease death, myocardial infarction, ischemic stroke or coronary revascularization were lower with obicetrapib compared to placebo, as also was 3-component MACE for coronary heart disease death, myocardial infarction or coronary revascularization.

In the overall pooled analysis, the incidence of cardiovascular events was lower in the obicetrapib group, although this failed to meet statistical significance (HR 95% CI, 0.77, 0.54-1.11, P=0.16). In contrast, the lower rate of coronary events (HR 95% CI, 0.68, 0.46-1.00, P=0.048) did meet statistical significance. Additional analyses demonstrated that obicetrapib treatment was associated with a lower rate of both cardiovascular (HR 95% CI, 0.60, 0.37-0.99, P=0.04) and coronary (HR 95% CI, 0.45, 0.26-0.77, P=0.003) adverse events, when examined in the period beyond 6 months after randomization.

## Claims

1. Pharmaceutical composition comprising obicetrapib or a pharmaceutically acceptable salt, hydrate, or solvate thereof, for use in a method of reducing the risk and/or incidence of a major adverse cardiovascular event (MACE) in 3-composite MACE (MACE3) or in 4-composite MACE (MACE4) in a subject having heterozygous familial hypercholesterolemia (HeFH) and/or atherosclerotic cardiovascular disease (ASCVD), the method comprising:
administering to the subject a therapeutically effective amount of a pharmaceutical composition comprising obicetrapib or a pharmaceutically acceptable salt, hydrate, or solvate thereof for at least 6 months,
wherein MACE3 comprises coronary heart disease death, nonfatal myocardial infarction and coronary revascularization; and MACE4 comprises coronary heart disease death, nonfatal myocardial infarction, ischemic stroke and coronary revascularization.

2. Pharmaceutical composition for use of claim 1, wherein the presence of HeFH is determined by genotyping.

3. Pharmaceutical composition for use of claims 1 or 2, wherein the presence of HeFH is determined by clinical assessment.

4. Pharmaceutical composition for use of any one of claims 1-3, wherein the presence of ASCVD is determined by the presence of at least one of the conditions selected from the group consisting of: coronary artery disease, cerebrovascular disease, and peripheral arterial disease.

5. Pharmaceutical composition for use of any one of claims 1-4, wherein the subject, prior to first administration of obicetrapib, has a fasting serum LDL-C equal to or greater than 70 mg/dL (1.81 mmol/L).

6. Pharmaceutical composition for use of any one of claims 1-5, wherein the subject, prior to first administration of obicetrapib, is on maximally tolerated lipid-modifying therapy, preferably wherein the maximally tolerated lipid-modifying therapy comprises at least one treatment selected from the group consisting of: a statin at a maximally tolerated stable dose, ezetimibe, bempedoic acid, a proprotein convertase subtilisin/kexin type 9 (PCSK9)-targeted therapy, a fibrate other than gemfibrozil, and combinations thereof.

7. Pharmaceutical composition for use of claim 6, wherein the subject, prior to first administration of obicetrapib, is on high-intensity statin therapy (HIS).

8. Pharmaceutical composition for use of claim 7, wherein the high-intensity statin therapy (HIS) is atorvastatin at 40 mg or 80 mg or rosuvastatin at 20 mg or 40 mg.

9. Pharmaceutical composition for use of any one of claims 1-8, wherein the subject, prior to the first administration of obicetrapib, is not diabetic.

10. Pharmaceutical composition for use of any one of claims 1-8, wherein the subject, prior to the first administration of obicetrapib, is normoglycemic.

11. Pharmaceutical composition for use of any one of claims 1-8, wherein the subject, prior to the first administration of obicetrapib, is diabetic.

12. Pharmaceutical composition for use of any one of claims 1-11, wherein obicetrapib or a pharmaceutically acceptable salt, hydrate, or solvate thereof, is administered orally at a dose of 10 mg once daily.

13. Pharmaceutical composition for use of claim 12, wherein obicetrapib or a pharmaceutically acceptable salt, hydrate, or solvate thereof is administered daily for at least 9 months, preferably for at least 12 months, more preferably for at least 24 months.

14. Pharmaceutical composition for use of any one of claims 1-13, wherein a calcium salt of obicetrapib is administered to the subject, preferably obicetrapib hemicalcium, more preferably amorphous obicetrapib hemicalcium.

15. Pharmaceutical composition for use of any one of claims 1-14, wherein the pharmaceutical composition is a solid dosage form, preferably a tablet, more preferably a tablet that further comprises a film coating.

## Patentansprüche

1. Pharmazeutische Zusammensetzung, die Obicetrapib oder ein pharmazeutisch verträgliches Salz, Hydrat oder Solvat davon umfasst, zur Verwendung in einem Verfahren zur Verringerung des Risikos und/oder der Inzidenz eines schwerwiegenden unerwünschten kardiovaskulären Ereignisses (MACE) bei 3-Komponenten-MACE (MACE3) oder bei 4-Komponenten-MACE (MACE4) bei einem Subjekt, das heterozygote familiäre Hypercholesterinämie (HeFH) und/oder atherosklerotische Herz-Kreislauf-Erkrankung (ASCVD) aufweist, wobei das Verfahren umfasst:
Verabreichung an das Subjekt eine therapeutisch wirksame Menge einer pharmazeutischen Zusammensetzung, die Obicetrapib oder ein pharmazeutisch verträgliches Salz, Hydrat oder Solvat davon umfasst, über mindestens 6 Monate,
wobei MACE3 koronaren Herzkrankheitstod, nicht-tödlichen Myokardinfarkt und koronare Revaskularisation umfasst; und MACE4 koronaren Herzkrankheitstod, nicht-tödlichen Myokardinfarkt, ischämischen Schlaganfall und koronare Revaskularisation umfasst.

2. Pharmazeutische Zusammensetzung zur Verwendung nach Anspruch 1, wobei das Vorhandensein von HeFH durch Genotypisierung bestimmt wird.

3. Pharmazeutische Zusammensetzung zur Verwendung nach Anspruch 1 oder 2, wobei das Vorhandensein von HeFH durch klinische Beurteilung bestimmt wird.

4. Pharmazeutische Zusammensetzung zur Verwendung nach einem der Ansprüche 1-3, wobei das Vorhandensein einer ASCVD durch das Vorhandensein mindestens einer der Erkrankungen bestimmt wird, ausgewählt aus der Gruppe, bestehend aus: koronarer Herzkrankheit, zerebrovaskulärer Erkrankung und peripherer arterieller Verschlusskrankheit.

5. Pharmazeutische Zusammensetzung zur Verwendung nach einem der Ansprüche 1-4, wobei das Subjekt vor der ersten Verabreichung von Obicetrapib einen Nüchternserum-LDL-C von gleich zu oder größer als 70 mg/dl (1,81 mmol/l) aufweist.

6. Pharmazeutische Zusammensetzung zur Verwendung nach einem der Ansprüche 1-5, wobei das Subjekt vor der ersten Verabreichung von Obicetrapib in einer maximal verträglichen lipidsenkenden Therapie ist, bevorzugt wobei die maximal verträgliche lipidsenkende Therapie mindestens eine Behandlung umfasst, ausgewählt aus der Gruppe bestehend aus: einem Statin in einer maximal verträglichen stabilen Dosis, Ezetimib, Bempedoinsäure, eine gegen Proprotein-Convertase-Subtilisin/Kexin Typ 9 (PCSK9) gerichtete Therapie, ein Fibrat außer Gemfibrozil und Kombinationen davon.

7. Pharmazeutische Zusammensetzung zur Verwendung nach Anspruch 6, wobei das Subjekt vor erster Verabreichung von Obicetrapib in hochintensiver Statintherapie (HIS) ist.

8. Pharmazeutische Zusammensetzung zur Verwendung nach Anspruch 7, wobei die hochintensive Statintherapie (HIS) Atorvastatin bei 40 mg oder 80 mg oder Rosuvastatin bei 20 mg oder 40 mg ist.

9. Pharmazeutische Zusammensetzung zur Verwendung nach einem der Ansprüche 1-8, wobei das Subjekt vor der ersten Verabreichung von Obicetrapib nicht diabetisch ist.

10. Pharmazeutische Zusammensetzung zur Verwendung nach einem der Ansprüche 1-8, wobei das Subjekt vor der ersten Verabreichung von Obicetrapib normoglykämisch ist.

11. Pharmazeutische Zusammensetzung zur Verwendung nach einem der Ansprüche 1-8, wobei das Subjekt vor der ersten Verabreichung von Obicetrapib diabetisch ist.

12. Pharmazeutische Zusammensetzung zur Verwendung nach einem der Ansprüche 1-11, wobei Obicetrapib oder ein pharmazeutisch verträgliches Salz, Hydrat oder Solvat davon oral in einer Dosis von 10mg einmal täglich verabreicht wird.

13. Pharmazeutische Zusammensetzung zur Verwendung nach Anspruch 12, wobei Obicetrapib oder ein pharmazeutisch verträgliches Salz, Hydrat oder Solvat davon täglich für mindestens 9 Monate, bevorzugt für mindestens 12 Monate, mehr bevorzugt für mindestens 24 Monate verabreicht wird.

14. Pharmazeutische Zusammensetzung zur Verwendung nach einem der Ansprüche 1-13, wobei ein Calciumsalz von Obicetrapib dem Subjekt verabreicht wird, bevorzugt Obicetrapib-Hemicalcium, mehr bevorzugt amorphes Obicetrapib-Hemicalcium.

15. Pharmazeutische Zusammensetzung zur Verwendung nach einem der Ansprüche 1-14, wobei die pharmazeutische Zusammensetzung eine feste Darreichungsform ist, bevorzugt eine Tablette, mehr bevorzugt eine Tablette, die ferner eine Filmbeschichtung umfasst.

## Revendications

1. Composition pharmaceutique comprenant de l'obicetrapib ou un sel, un hydrate ou un solvate pharmaceutiquement acceptable de celui-ci, destinée à être utilisée dans un procédé visant à réduire le risque et/ou l'incidence d'un événement cardiovasculaire indésirable majeur (MACE) dans le cadre d'un MACE composite à 3 critères (MACE3) ou dans un MACE composite à 4 critères (MACE4) chez un sujet ayant une hypercholestérolémie familiale hétérozygote (HeFH) et/ou une maladie cardiovasculaire athérosclérotique (ASCVD), le procédé comprenant :
l'administration au sujet d'une quantité thérapeutiquement efficace d'une composition pharmaceutique comprenant de l'obicetrapib ou un sel, un hydrate ou un solvate pharmaceutiquement acceptable de celui-ci pendant au moins 6 mois,
où le MACE3 comprend le décès par maladie coronarienne, l'infarctus du myocarde non fatal et la revascularisation coronarienne ; et le MACE4 comprend le décès par maladie coronarienne, l'infarctus du myocarde non fatal, l'accident vasculaire cérébral ischémique et la revascularisation coronarienne.

2. Composition pharmaceutique destinée à être utilisée selon la revendication 1, où la présence de HeFH est déterminée par génotypage.

3. Composition pharmaceutique destinée à être utilisée selon les revendications 1 ou 2, où la présence de HeFH est déterminée par évaluation clinique.

4. Composition pharmaceutique destinée à être utilisée selon l'une quelconque des revendications 1 à 3, où la présence d'ASCVD est déterminée par la présence d'au moins l'une des affections choisies parmi le groupe constitué de : la maladie coronarienne, la maladie vasculaire cérébrale et la maladie artérielle périphérique.

5. Composition pharmaceutique destinée à être utilisée selon l'une quelconque des revendications 1 à 4, où le sujet, avant la première administration d'obicetrapib, présente un taux sérique de LDL-C à jeun égal ou supérieur à 70 mg/dl (1,81 mmol/l).

6. Composition pharmaceutique destinée à être utilisée selon l'une quelconque des revendications 1 à 5, où le sujet, avant la première administration d'obicetrapib, suit un traitement hypolipidémiant à la dose maximale tolérée, de préférence où le traitement hypolipidémiant à la dose maximale tolérée comprend au moins un traitement choisi dans le groupe constitué par : une statine à une dose stable maximale tolérée, l'ézétimibe, l'acide bempédoïque, un traitement ciblant la proprotéine convertase subtilisine/kexine de type 9 (PCSK9), un fibrate autre que le gemfibrozil, et des combinaisons de ceux-ci.

7. Composition pharmaceutique destinée à être utilisée selon la revendication 6, où le sujet, avant une première administration d'obicetrapib, suit un traitement par statine à haute intensité (HIS).

8. Composition pharmaceutique destinée à être utilisée selon la revendication 7, où le traitement par statine à haute intensité (HIS) consiste en de l'atorvastatine à 40 mg ou 80 mg, ou en de la rosuvastatine à 20 mg ou 40 mg.

9. Composition pharmaceutique destinée à être utilisée selon l'une quelconque des revendications 1 à 8, où le sujet, avant la première administration d'obicetrapib, n'est pas diabétique.

10. Composition pharmaceutique destinée à être utilisée selon l'une quelconque des revendications 1 à 8, où le sujet, avant la première administration d'obicetrapib, est normoglycémique.

11. Composition pharmaceutique destinée à être utilisée selon l'une quelconque des revendications 1 à 8, où le sujet avant la première administration d'obicetrapib, est diabétique.

12. Composition pharmaceutique destinée à être utilisée selon l'une quelconque des revendications 1 à 11, où l'obicetrapib, ou un sel, un hydrate ou un solvate pharmaceutiquement acceptable de celui-ci, est administré par voie orale à une dose de 10 mg une fois par jour.

13. Composition pharmaceutique destinée à être utilisée selon la revendication 12, où l'obicetrapib ou un sel, un hydrate ou un solvate pharmaceutiquement acceptable de celui-ci est administré quotidiennement pendant au moins 9 mois, de préférence pendant au moins 12 mois, plus préférablement pendant au moins 24 mois.

14. Composition pharmaceutique destinée à être utilisée selon l'une quelconque des revendications 1 à 13, où un sel de calcium de l'obicetrapib est administré au sujet, de préférence de l'hémicalcium d'obicetrapib, plus préférablement de l'hémicalcium d'obicetrapib amorphe.

15. Composition pharmaceutique destinée à être utilisée selon l'une quelconque des revendications 1 à 14, où la composition pharmaceutique se présente sous une forme posologique solide, de préférence un comprimé, plus préférablement un comprimé qui comprend en outre un pelliculage.
